(19) Europäisches Patentamt
European Patent Office
Office européen des brevets

(11) EP 2 931 749 B1

(12) **FASCICULE DE BREVET EUROPEEN**

(45) Date de publication et mention de la délivrance du brevet:
**24.04.2019 Bulletin 2019/17**

(21) Numéro de dépôt: **13818329.8**

(22) Date de dépôt: **17.12.2013**

(51) Int Cl.:
***C07K 16/22*** *(2006.01)* ***C07K 16/24*** *(2006.01)*
***A61K 39/395*** *(2006.01)* ***A61P 29/00*** *(2006.01)*

(86) Numéro de dépôt international:
**PCT/FR2013/053120**

(87) Numéro de publication internationale:
**WO 2014/096672 (26.06.2014 Gazette 2014/26)**

(54) **UTILISATION D'ANTICORPS MONOCLONAUX POUR LE TRAITEMENT DE L'INFLAMMATION ET D'INFECTIONS BACTERIENNES**

VERWENDUNG MONOKLONALER ANTIKÖRPER ZUR BEHANDLUNG VON ENTZÜNDUNGEN UND BAKTERIELLEN INFEKTIONEN

USE OF MONOCLONAL ANTIBODIES FOR THE TREATMENT OF INFLAMMATION AND BACTERIAL INFECTIONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.12.2012 FR 1262178**
**15.03.2013 FR 1352362**

(43) Date de publication de la demande:
**21.10.2015 Bulletin 2015/43**

(60) Demande divisionnaire:
**19159751.7**

(73) Titulaire: **Laboratoire Français du Fractionnement et des Biotechnologies Société Anonyme**
**91940 Les Ulis (FR)**

(72) Inventeur: **DE ROMEUF, Christophe**
**F-59130 Lambersart (FR)**

(74) Mandataire: **Grosset-Fournier, Chantal Catherine et al**
**Grosset-Fournier & Demachy**
**54, rue Saint-Lazare**
**75009 Paris (FR)**

(56) Documents cités:
**WO-A1-2008/145866**
**WO-A1-2010/088444**
**WO-A2-2013/011076**
**US-A1- 2010 234 574**
**US-A1- 2012 088 905**

- **STROHL ET AL: "Optimization of Fc-mediated effector functions of monoclonal antibodies", CURRENT OPINION IN BIOTECHNOLOGY, LONDON, GB, vol. 20, no. 6, 1 décembre 2009 (2009-12-01), pages 685-691, XP026778879, ISSN: 0958-1669, DOI: 10.1016/J.COPBIO.2009.10.011 [extrait le 2009-11-04]**
- **CHAN ANDREW C ET AL: "Therapeutic antibodies for autoimmunity and inflammation", THE JOURNAL OF IMMUNOLOGY, NATURE PUB. GROUP, vol. 10, no. 5, 1 mai 2010 (2010-05-01), pages 301-316, XP002665072, ISSN: 1474-1733, DOI: 10.1038/NRI2761**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La demande ou l'invention concerne l'utilisation d'anticorps monoclonaux pour le traitement de l'inflammation, quelle qu'en soit l'origine.

L'inflammation, réponse normale de défense suite à une agression telle qu'une infection, une brûlure, une allergie..., est une réponse immunitaire stéréotypée. L'inflammation se traduit par une réponse immédiate et transitoire mettant en jeu un ensemble de réactions cellulaires et moléculaires, locales et périphériques, déclenchées à partir du foyer de l'agression, dans le but de circonscrire l'agression, puis de la résoudre. L'inflammation est donc un processus de prévention suivi d'une réparation, étapes nécessaires au retour de la fonction normale du tissu agressé.

Cette manifestation inflammatoire est la résultante de la libération de messagers moléculaires, appelés facteurs inflammatoires moléculaires, ou substances chimiques, appelés facteurs inflammatoires chimiques, qui vont à leur tour mobiliser des réponses générales de l'organisme qui sont autant de signaux systémiques. On distingue des réponses inflammatoires septiques (bactéries, virus, parasites) et des réponses stériles issues d'un syndrome métabolique comme le diabète, les hypercholestérolémies... L'intensité de l'inflammation dépend de l'agent infectieux et de la localisation du foyer, et donc de la nature du tissu. La réaction peut être latente puis être amplifiée par d'autres agents.

Le phénomène inflammatoire normal, ou inflammation aigue, se décompose en deux phases principales : l'initiation et la progression.

La phase d'initiation est induite par les cellules infectées par les virus ou les bactéries, par la présence de corps étrangers ou l'accumulation de molécules toxiques (radicaux, lipides, cholestérol...). Les premières réactions libèrent des messages qui diffusent en induisant des signaux de mort cellulaire voire de nécrose. Les cibles cellulaires sont les cellules endothéliales qui adoptent un changement morphologique par chimiotactisme laissant infiltrer des cellules du plasma sanguin.

La phase de progression de l'inflammation périphérique se caractérise en effet par une activation des terminaisons nerveuses qui provoque une vasodilatation facilitant la diffusion des molécules dans l'espace extracellulaire.

Les cellules infiltrées sont les mastocytes dégranulés, les monocytes, les macrophages, les polynucléaires neutrophiles, les lymphocytes. Ces cellules s'agrègent en raison de la forte production de facteurs chimioattractants. Les molécules chimiotactiques sont captées par des récepteurs qui induisent un changement des propriétés migratoires des cellules.

Les macrophages activés libèrent un large spectre de médiateurs constitués de glycoprotéines de faible taille dénommées cytokines comme l'interleukine-1 (IL-1) et le TNF *(tumor necrosis factor)* ou cachectine. Ces médiateurs, dont l'action est pléiotropique, orchestrent les mécanismes qui contribuent à la mise en place d'une réponse élargie de l'inflammation. L'IL-1 et le TNF$\alpha$ agissent sur les cellules stromales, les fibroblastes, les cellules musculaires lisses et provoquent la libération d'une seconde vague de cytokines et de molécules attractives des monocytes en activant d'autres cellules quiescentes.

L'inflammation aigue est donc un processus d'urgence de l'organisme en cas d'altération, mais cette inflammation peut aussi se retourner contre l'organisme si elle est activée de manière chronique.

L'inflammation chronique correspond à un échec de l'inflammation aiguë. La persistance de l'inflammation va être responsable de séquelles anatomiques et fonctionnelles qui font la gravité des maladies inflammatoires chroniques.

En effet, la persistance de la sécrétion de cytokines pro inflammatoires telles que le TNF$\alpha$ ou certaines interleukines maintenant l'inflammation peut induire une dégradation tissulaire et cellulaire.

Le mécanisme de la chronicité n'est pas toujours compris. Il peut s'agir de la persistance de la substance pathogène. Mais il est aussi possible que cette inflammation se perpétue en l'absence de tout agent pathogène.

Cette réaction inflammatoire accompagne de nombreuses grandes pathologies chroniques. La chronicité de l'inflammation et sa localisation à plusieurs organes est à l'origine du concept des maladies systémiques, maladies au cours desquelles l'autoimmunité joue un rôle important dans l'entretien de l'inflammation : lupus érythémateux disséminé, polyarthrite rhumatoïde, maladie de Gougerot-Sjögren, maladie de Crohn, colite ulcéreuse, maladie de Basedow (hyperthyroïdie), thyroïdite chronique de Hashimoto (hypothyroïdie), syndrome de Goodpasture, pemphigus, myasthénie, diabète par insulinorésistance, anémie hémolytique auto-immune, purpura thrombocytopénique auto-immun, sclérodermie, polymyosite et dermatomyosite, anémie de Biermer, glomérulonéphrite, maladie de Wegener, maladie de Horton, périartérite noueuse et le syndrome de Churg et Strauss, maladie de Still, polychondrite atrophiante, maladie de Behçet, sclérose en plaques, spondylarthrite. S'agissant des infections bactériennes, elles peuvent être causées par des toxines, c'est-à-dire des substances toxiques solubles produites par des bactéries, également par des champignons, des protozoaires ou des vers.

Les toxines bactériennes peuvent agir à des taux très faibles et agissent soit au niveau membranaire, soit sur des cibles intracellulaires, et sont parmi les substances biologiques les plus actives. Lesdites toxines peuvent être réparties en deux grandes catégories : les exotoxines et les endotoxines. Les exotoxines sont des protéines produites par des bactéries et sécrétées dans le milieu environnant tandis que les endotoxines sont des liposaccharides, à savoir des constituants de la membrane externe de la paroi des bactéries à gram négatif libérées à la suite de la lyse bactérienne

La prophylaxie ou le traitement de telles infections au moyen d'anticorps dirigés contre lesdites toxines est bien connue de l'homme du métier.

Un premier moyen préventif est d'immuniser les individus avec des toxines inactivées et immunogènes (les anatoxines), afin de permettre la production par l'individu de lymphocytes B mémoires susceptibles d'être activés et d'intervenir rapidement en cas d'infection bactérienne susceptible de libérer la toxine.

L'homme du métier connait les moyens pour traiter lesdites infections. Il existe des anticorps neutralisants dirigés contre les toxines, par exemple, tels que ceux décrits dans les documents FR 55671 et EP 0 562 132 pour traiter le tétanos, ou les documents US7700738 et US20100222555 pour traiter le botulisme. Pour exemple, le traitement actuel d'une infection bactérienne à *Clostridium tetani* repose sur l'administration d'un sérum anti-tétanique constitué d'immunoglobulines humaines anti-tétanos, visant à apporter au patient des anticorps dans l'attente qu'il en produise, de l'anatoxine tétanique visant à stimuler son système immunitaire afin qu'il produise ses propres anticorps, d'un antibiotique ainsi que de myorelaxants et de sédatifs.

Un anticorps est dit neutralisant lorsqu'il bloque l'effet de la toxine, et notamment sa fixation à sa cible et/ou son entrée dans la cellule cible.

Néanmoins, de tels anticorps ne permettent pas d'éliminer la toxine et lorsque celle-ci est liée à sa cellule cible, les anticorps deviennent inopérants.

WO 2008/145866 A1 est relatif à un ensemble de moyens pour le traitement d'une pathologie maligne, d'une maladie auto-immune ou d'une maladie infectieuse. US 8 324 351 B2 et US 2012/0088905 A1 sont relatifs à des variants Fc qui présentent une liaison altérée au FcRn. WO 2010/088444 A1 est relatif à des anticorps anti-IL-6 humain, qui présentent une demi-vie plus longue et à leur utilisation en oncologie, pour le traitement de maladies auto-immunes et pour le traitement de maladies inflammatoires. Strohl 2009 (Current Opinion in Biotechnology 20 :685-691) est relatif à l'optimisation de fonctions effectrices d'anticorps monoclonaux médiées par Fc. WO 2013/011076 A2 est relatif à des protéines se liant à antigène, qui présentent une liaison accrue au FcRn.

Aussi, il existe à ce jour un réel besoin de fournir un traitement permettant de limiter les effets de l'inflammation chronique et de limiter les effets de l'infection bactérienne causant la libération d'une toxine dans un organisme.

Par conséquent, l'un des buts de la demande ou de l'invention est de fournir des moyens de traiter les organismes infectés par une toxine ou de prévenir les effets délétères de ladite toxine.

Un autre but de la demande ou de l'invention est de fournir un moyen d'éliminer les toxines bactériennes de l'organisme contaminé. Un autre but de la demande ou de l'invention est de fournir une composition permettant d'éradiquer la toxine et la bactérie ou le microorganisme qui la produit.

Par conséquent, l'un des buts de la demande ou de l'invention est de fournir des moyens de prévenir et de soigner les organismes soumis à une inflammation.

Un autre but de la demande ou de l'invention est de fournir un moyen d'éliminer les cytokines pro inflammatoires.

La demande ou l'invention concerne donc une composition comprenant des anticorps monoclonaux dirigés soit contre une cytokine pro-inflammatoire circulante, soit contre une toxine bactérienne circulante, lesdits anticorps ayant une forte affinité pour le récepteur FcγRIIIa (CD16), pour son utilisation dans le cadre de la prévention ou du traitement des phases précoces de l'inflammation.

L'invention est relative à une composition comprenant des anticorps monoclonaux dirigés contre une cytokine pro-inflammatoire circulante, lesdits anticorps ayant une affinité au moins égale à $2x10^7$ $M^{-1}$ pour le récepteur FcγRIIIa (CD16) et dans laquelle le taux de fucose de l'ensemble des anticorps de ladite composition est inférieur à 60%, pour son utilisation dans le cadre de la prévention ou du traitement de l'inflammation, notamment dans laquelle lesdits anticorps ont une affinité au moins égale à $2x10^8$ $M^{-1}$ ou $2x10^9$ $M^{-1}$, telle que déterminée par l'analyse Scatchard ou la technologie BIAcore (Label-free surface plasmon resonance based technology).

Les anticorps sont sécrétés par les cellules du système immunitaire, les plasmocytes (aussi appelés les lymphocytes B sécréteurs) et constituent les immunoglobulines principales du sang. Par la suite, les termes « immunoglobuline » et « anticorps » sont équivalents.

Un anticorps se lie à l'antigène pour lequel il est spécifique. L'antigène peut être soluble ou membranaire et est constitué soit par un élément étranger à l'organisme (antigène bactérien, viral, ...) soit par un élément constitutif de l'organisme (auto-antigène).

Selon un mode de réalisation de la demande ou de l'invention, l'antigène est soit une cytokine pro-inflammatoire circulante, soit une toxine bactérienne circulante.

La structure des immunoglobulines (Ig) est bien connue de l'homme du métier. Il s'agit de tétramères constitués de deux chaînes lourdes d'environ 50 kDa chacune (dites les chaînes H pour Heavy) et de deux chaînes légères d'environ 25 kDa chacune (dites les chaînes L pour Light), liées entre elles par des ponts disulfures intra et intercaténaires.

Chaque chaîne est constituée, en position N-terminale, d'une région ou domaine variable, dit VL pour la chaîne légère, VH pour la chaîne lourde, et en position C-terminale, d'une région constante, constitué d'un seul domaine dit CL pour la chaîne légère et de trois ou quatre domaines nommés CH1, CH2, CH3, CH4, pour la chaîne lourde.

Seules les IgM et les IgE possèdent le domaine CH4.

Les domaines constants sont codés par les gènes C et les domaines variables sont codés par les gènes V-J pour la chaîne légère et V-D-J pour la chaîne lourde.

L'assemblage des chaines qui composent un anticorps permet de définir une structure tridimensionnelle caractéristique en Y, où :

- la base du Y correspond à la région constante Fc qui est reconnue par le complément et les récepteurs Fc (RFc) afin de médier les fonctions effectrices de la molécule, et
- les extrémités des bras du Y correspondent à l'assemblage respectif de la région variable d'une chaîne légère et de la région variable d'une chaîne lourde, lesdites extrémités déterminant la spécificité de l'anticorps pour l'antigène.

Plus précisément, il existe cinq isotypes de chaînes lourdes (gamma, alpha, mu, delta et epsilon) et deux isotypes de chaînes légères (kappa et lambda, les chaînes lambda étant elles-mêmes divisées en deux types : lambda 1 et lambda 2). C'est la chaîne lourde qui détermine la classe d'immunoglobuline. Il y a ainsi cinq classes d'Ig : IgG pour l'isotype gamma, IgA pour l'isotype Alpha, IgM pour l'isotype Mu, IgD pour l'isotype Delta et IgE pour l'isotype Epsilon.

Les chaînes légères kappa et lambda sont partagées par toutes les classes et sous-classes. Chez l'homme, la proportion de kappa et lambda produite se situe dans un rapport de 2 pour 1.

De façon plus précise, au niveau des domaines VH et VL, la variabilité de séquences n'est pas distribuée de manière égale. En effet, les régions variables sont constituées de quatre régions très peu variables nommées charpentes ou *Framework* (FR1 à FR4) entre lesquelles s'insèrent trois régions hypervariables ou *Complementary Determining Regions* (CDR1 à CDR3). Chaque VH et VL est composée de trois CDRs et quatre FRs disposés depuis l'amine terminal (Nterminal) jusqu'au carboxy terminal (Cterminal) dans l'ordre suivant : FR1, CDR1, FR2, CDR2, FR3, CDR3, FR4. Les régions variables de la chaîne lourde et de la chaîne légère ont un domaine de liaison qui interagit avec l'antigène. Les régions constantes des anticorps peuvent médier la liaison de l'immunoglobuline, aux tissus de l'hôte ou facteurs, incluant les diverses cellules du système immunitaire (par exemple les cellules effectrices), et le premier composant (C1q) du système du complément classique. La formation d'une molécule d'anticorps fonctionnel mature peut être accomplie quand deux protéines sont exprimées en quantité stoechiométrique et s'assemblent spontanément dans la bonne configuration.

Pour de plus amples informations sur la structure et les propriétés des différentes classes d'immunoglobulines, l'homme du métier se référera à Daniel P. Stites *et al., « Basic and Clinical Immunology »,* 8ème edition, Appleton & Lange, Norwalk, Conn., 1994, page 71 et Chapitre 6.

Le terme « anticorps » désigne également un fragment de liaison à l'antigène. Les procédés pour faire des anticorps et des fragments de liaison à l'antigène sont bien connus par l'homme du métier (voir e.g., Sambrook et al, "Molecular Cloning: A Laboratory Manual" (2nd Ed.), Cold Spring Harbor Laboratory Press (1989); Lewin, "Genes IV", Oxford University Press, New York, (1990), et Roitt et al., "Immunology" (2nd Ed.), Gower Medical Publishing, London, New York (1989), WO2006/040153, WO2006/122786, et WO2003/002609).

Selon la demande ou l'invention, un « fragment de liaison à l'antigène » d'un anticorps fait référence à une ou plusieurs portions d'un anticorps qui conserve la capacité à se lier spécifiquement à un antigène.

Il a été montré que la fonction de liaison à l'antigène d'un anticorps peut être effectuée par des fragments de la longueur totale de l'anticorps. Des exemples de fragments de liaison compris sous le terme « fragment de liaison à l'antigène » d'un anticorps inclut : (i) un fragment Fab, un fragment monovalent consistant en les domaines VL, VH, CL et CH1 ; (ii) un fragment F(ab')2, un fragment bivalent comprenant deux fragments Fab liés par un pont disulfure à la région charnière ; (iii) un fragment Fd consistant en les domaines VH et CH1 ; (iv) un fragment Fv consistant en les domaines VL et VH d'un seul bras d'un anticorps, (v) un fragment dAb (Ward et al., (1989) Nature 341:544-546) qui consiste en un domaine VH ; et (vi) une région de détermination de la complémentarité isolée (CDR). De plus, bien que les deux domaines du fragment Fv, V et VH, soient codés par des gènes séparés, ils peuvent être joints, par des techniques de recombinaison, via une liaison synthétique qui permet de les constituer en une seule chaîne de protéines dans laquelle les régions VH et VL s'associent en paire pour former des molécules monovalentes (connues comme des chaînes simples Fv (scFv) ; voir par exemple Bird et al. (1988) Science 242:423-426; and Huston et al. (1988) Proc. Natl. Acad. Sci. USA 85:5879-5883). De telles simples chaines d'anticorps sont également comprises dans le terme « portion de liaison à l'antigène » d'un anticorps. Ces fragments d'anticorps sont obtenus en utilisant des procédés conventionnels, par exemple les procédés de fragmentation protéolytique, comme décrit dans J. Goding, Monoclonal Antibodies: Principles and Practice, pp 98-118 (N.Y. Academic Press 1983), de même que d'autres techniques connues de l'homme de l'art. Les fragments sont analysés pour leur utilité de la même manière que les anticorps entiers.

Selon un aspect particulier de la demande ou de l'invention, les anticorps sont de l'isotype IgG, Iga ou IgD. Selon encore un autre aspect, les anticorps sont sélectionnés dans le groupe constitué par IgG1, IgG2, IgG3, IgG4, IgM, IgA1, IgA2, IgAsec, IgD, IgE ou a les domaines constants et/ou variables des susdites immunoglobulines.

Selon un autre aspect les anticorps sont des anticorps bispécifiques ou des anticorps multispécifiques.

Selon un mode alternatif de la demande ou de l'invention, les anticorps de la présente invention peuvent être

modifiés sous la forme d'un anticorps bispécifique ou d'un anticorps multispécifique.

Selon la demande ou l'invention, le terme « anticorps bispécifique », inclut n'importe quel agent, par exemple, une protéine, un peptide, ou un complexe protéique ou un complexe peptidique, qui a deux spécificités de liaison différentes, se liant ou interagissant avec (a) un antigène de surface cellulaire et (b) un récepteur Fc sur la surface d'une cellule effectrice.

Le terme « anticorps multispécifique » inclut n'importe quel agent, par exemple, une protéine, un peptide, ou un complexe protéique ou un complexe peptidique, qui a plus de deux spécificités de liaison différentes, se liant ou interagissant avec (a) un antigène de surface cellulaire et (b) un récepteur Fc sur la surface d'une cellule effectrice, et (c) au moins un autre composant. La demande ou l'invention inclut donc, mais n'est pas limitée, aux anticorps bispécifiques, aux anticorps trispécifiques, aux anticorps tétraspécifiques, et autres anticorps multispécifiques qui sont dirigés contre des antigènes de surface cellulaire, et contre des récepteurs sur les cellules effectrices. Le terme « anticorps bispécifiques » inclut également les di-anticorps. Les di-anticorps sont des anticorps bi-spécifiques, bivalents dans lesquels les domaines VH et VL sont exprimés sur une seule chaîne polypeptidique, mais en utilisant une liaison qui est trop courte pour permettre l'association entre les deux domaines de la même chaîne, forçant ainsi les domaines à s'associer avec les domaines complémentaires d'une autre chaîne et créant ainsi deux sites de liaison à l'antigène (voir Holliger, P., et al. (1993) Proc. Natl. Acad. Sci. USA 90:6444-6448; Poijak, R.J., et al. (1994) Structure 2:1121-1123).

Le terme anticorps inclut également différents types d'anticorps, par exemple les anticorps recombinants, les anticorps murins, les anticorps chimériques, les anticorps humanisés, les anticorps humains, les anticorps monoclonaux ou un mélange de ces anticorps.

Selon un aspect particulier, les anticorps sont des anticorps recombinants. Le terme « anticorps recombinants », tel qu'utilisé ici inclut les anticorps qui sont préparés, exprimés, créés, ou isolés par des moyens recombinants, tels des anticorps isolés d'un animal qui est transgénique pour les gènes d'immunoglobuline d'une autre espèce, les anticorps exprimés en utilisant un vecteur d'expression recombinant transfecté dans une cellule hôte, les anticorps isolés d'une bibliothèque d'anticorps recombinants combinatoires, ou les anticorps préparés, exprimés, créés, ou isolés par n'importe quel autre moyen qui implique l'épissage des séquences de gènes d'immunoglobuline dans d'autres séquences d'ADN.

Par « anticorps murins », on entend des anticorps contenant uniquement des séquences appartenant à toutes espèces de souris. Comme exemple d'un tel anticorps on peut citer l'anticorps anti-CD3 (Orthoclone OKT3®, muromonab-CD3) qui est le premier anticorps monoclonal murin admis pour l'usage thérapeutique chez l'homme.

Par « anticorps chimériques », on entend des anticorps dont les séquences des régions variables des chaînes légères et des chaînes lourdes appartiennent à une espèce différente de celle des séquences des régions constantes des chaînes légères et des chaînes lourdes.

Aux fins de la demande ou de l'invention, les séquences des régions variables des chaînes lourdes et légères sont préférentiellement d'espèce murine tandis que les séquences des régions constantes des chaînes lourdes et légères appartiennent à une espèce non-murine. A cet égard, pour les régions constantes, toutes les familles et espèces de mammifères non-murins sont susceptibles d'être utilisées, et en particulier l'homme, le singe, les muridés (sauf la souris), les suidés, les bovidés, les équidés, les félidés, les canidés ou encore les oiseaux, cette liste n'étant pas exhaustive.

De façon préférée, les anticorps chimériques selon la demande ou l'invention contiendront des séquences des régions constantes des chaînes lourdes et légères d'anticorps humains et les séquences des régions variables des chaînes lourdes et légères d'anticorps murins. Pour exemples de tels anticorps chimériques souris/humain, on peut citer le rituximab (Mabthera®), un anti-CD20 et le cétuximab (Erbitux®), un anti-EGFR.

Selon la demande ou l'invention, le terme « anticorps humanisé » fait référence à un anticorps qui conserve uniquement les régions CDRs de liaison à l'antigène de l'anticorps parent, en association avec les régions charpentes (framework) humaines (voir Waldmann, 1991, Science 252:1657). Cela fait notamment référence à des anticorps dont tout ou partie des séquences des régions impliquées dans la reconnaissance de l'antigène (les régions hypervariables (*CDR : Complementarity Determining Region*) et parfois certains acides aminés des régions FR (régions *Framework*)) appartiennent à des séquences d'origine non-humaine tandis que les séquences des régions constantes et des régions variables non-impliquées dans la reconnaissance de l'antigène sont d'origine humaine. Comme exemple d'un tel anticorps, on peut citer le daclizumab (Zenapax®), le premier anticorps humanisé à avoir été utilisé en clinique.

De tels anticorps humanisés ou chimériques contenant les sites de liaison spécifiques de l'anticorps murin sont attendus pour avoir une immunogénicité réduite quand ils sont administrés *in vivo* pour le diagnostic, pour des applications prophylactiques ou thérapeutiques selon la demande ou l'invention.

Le terme « anticorps humains », tel qu'utilisé ici, inclut des anticorps ayant des régions variables et constantes dérivées des séquences d'immunoglobulines germinales humaines. Les anticorps humains peuvent également inclure des résidus acides aminés non codés par des séquences d'immunoglobulines germinales humaines (par exemple, des mutations introduites au hasard, ou par mutagénèse *in vitro* site-spécifique, ou par mutation somatique *in vivo*). Des anticorps humains sont générés en utilisant des souris transgéniques portant plutôt des parties du système immunitaire humain que celui de souris. Des anticorps monoclonaux pleinement humains peuvent également être préparés en immunisant des souris transgéniques avec de grandes parties des chaînes lourdes et légères d'immunoglobulines

humaines. Cf les brevets américains U.S. 5,591,669, 5,598,369, 5,545,806, 5,545,807, 6,150,584, et les références citées dedans. Ces animaux ont été génétiquement modifiés de façon à ce qu'il y ait une délétion fonctionnelle dans la production d'anticorps endogènes (par exemple murin). Les animaux sont également modifiés pour contenir toute ou une portion du locus de gène d'immunoglobuline germinale humaine de façon à ce que l'immunisation de ces animaux résulte dans la production d'anticorps pleinement humains dirigés contre l'antigène d'intérêt. Selon l'immunisation de ces souris (par exemple XenoMouse (Abgenix), souris HuMAb (Medarex/GenPharm)), les anticorps monoclonaux sont préparés selon les techniques d'hybridomes classiques. Ces anticorps monoclonaux ont des séquences d'acides aminés d'immunoglobuline humaine et ainsi ne provoquent pas de réponses d'anticorps humain anti-murins (HAMA) quand ils sont administrés à des humains. Les anticorps humains, comme n'importe quel anticorps peuvent être des anticorps monoclonaux.

Comme exemple d'un tel anticorps, on peut citer l'adalimumab (Humira®), un anti-TNF-$\alpha$, le premier anticorps humain à avoir été autorisé pour une utilisation clinique.

Aux fins de la demande ou de l'invention, les anticorps sont monoclonaux. Les anticorps monoclonaux ou les « compositions d'anticorps monoclonaux » font référence à des anticorps possédant une spécificité unique vis-à-vis d'un antigène en se liant à un seul épitope. Ils s'opposent aux anticorps polyclonaux qui sont des mélanges d'immunoglobulines isolées du sérum et qui reconnaissent une série d'épitopes différents sur l'antigène considéré.

Selon un autre aspect particulier, l'anticorps est un anticorps de longueur totale. Selon encore un autre aspect particulier, cet anticorps de longueur totale comprend une chaîne légère et une chaîne lourde.

L'affinité desdits anticorps peut être déterminée par plusieurs méthodes, dont la résonance plasmonique de surface (RPS), à l'aide d'un appareil type BIAcore 2000 (*Pharmacia Biosensor,* Upsala, Suède). Les documents Malmquist M., Current Opinion in Immunology, 5:282-286 (1993) ; Jönsson U et al., Biotechniques, 11:620-627 (1991) et Wu et al., Proc. Natl. Acad. Sci. USA, 95:6037-6042 (1998) décrivent ce type de mesure.

Par « affinité », on entend la somme des forces de liaison et de répulsion entre un épitope et un paratope. Cela représente la capacité qu'a un anticorps à se lier à un antigène. L'affinité est souvent exprimée par la constante d'affinité (Kd ou constante de dissociation à l'équilibre). La constante d'affinité est liée à la constante de dissociation (Koff ou kd) et à la constante d'association (Kon ou ka) par la relation Kd = Koff / Kon = kd / ka.

Par « constante de dissociation », on entend la constante de réaction associée à la dissociation d'un complexe antigène-anticorps.

Par « constante d'association », on entend la constante de réaction associée à l'association d'un complexe antigène-anticorps.

Les différents isotypes des immunoglobulines (IgM, IgA, IgE, IgD, IgG) diffèrent quant à leur activité biologique et leurs fonctions effectrices. Il existe également des différences d'activité entre les sous-classes des IgA (IgA1, IgA2) et des IgG (IgG1, IgG2, IgG3, IgG4). Ces différences dépendent des récepteurs auxquels les régions Fc des immunoglobulines se lient et de la distribution de ces récepteurs sur la membrane des cellules effectrices.

Par « récepteur », on entend les récepteurs au fragment Fc (FcR). Lesdits récepteurs sont Fc$\alpha$R (IgA) ; Fc$\gamma$RI, Fc$\gamma$RII, Fc$\gamma$RIII (IgG) ; Fc$\varepsilon$RI, Fc$\varepsilon$RII (IgE) ; Fc$\mu$R (IgM) ; Fc$\delta$R (IgD) ; pIgR (récepteur poly-Ig) et FcRn (récepteur Fc néonatal).

Les récepteurs aux IgG sont également appelés CD64 (Fc$\gamma$RI), CD32 (Fc$\gamma$RII) et CD16 (Fc$\gamma$RIII). Huit gènes codant pour les Fc$\gamma$R ont été identifiés chez l'homme, mais seulement cinq codent pour des récepteurs exprimés (Fc$\gamma$RIa, Fc$\gamma$RIIa, Fc$\gamma$RIIb, Fc$\gamma$RIIIa, Fc$\gamma$RIIIb). Tous sont des récepteurs activateurs des cellules effectrices, hormis le Fc$\gamma$RIIb qui lui est un récepteur inhibiteur de l'activation des cellules immunitaires (Muta T et al., Nature, 1994, 368:70-73).

Plus précisément, lesdits récepteurs de type I sont caractérisés par une forte affinité pour les immunoglobulines (Kd de $5.10^{-7}$ à $10^{-10}$ M) tandis que les types II et III sont caractéristiques des récepteurs de faible affinité (Kd inférieure à $10^{-7}$ M).

Pour plus d'informations sur les récepteurs Fc, voir Ravetch and Kinet, Annual Review of Immunology, vol 9:457-492 (1991).

Par « cellule effectrice », on entend toute cellule porteuse d'un récepteur Fc, telles que les lymphocytes, les monocytes, les neutrophiles, les cellules Natural Killer (NK), les éosinophiles, les basophiles, les mastocytes, les cellules dendritiques, les cellules de Langerhans et les plaquettes.

La région Fc est responsable des fonctions effectrices de l'anticorps, notamment des fonctions cytotoxiques.

C'est également cette région qui détermine la durée de demi-vie sérique de l'anticorps.

Par « fonction effectrice », on fait notamment référence à l'ADCC (*Antibody Dependant Cellular Cytotoxicity),* la CDC (*Complement Dependant Cytotoxicity*) et l'ADCP (*Antibody Dependent Cellular Phagocytosis).* Lesdites fonctions ont pour finalité d'éliminer les cellules cibles de l'organisme.

Par « cellule cible », on entend toute cellule porteuse d'antigènes. Lesdits antigènes peuvent être des substances étrangères à l'organisme (pathogènes) ou des molécules du « soi », dans le cadre de maladies auto-immunes par exemple.

L'ADCC est un mécanisme de défense de l'organisme médié par les cellules effectrices. Lesdites cellules

reconnaissent les cellules cibles recouvertes d'anticorps spécifiques. La liaison RFc-Fc active les cellules effectrices, qui vont alors détruire les cellules cibles par apoptose par exemple, à la suite de la libération par les granules cytoplasmiques de perforine et granzymes (Raghavan et al., Annu Rev Cell Dev Biol 12:181-220, 1996 ; Ravetch et al., Annu Rev Immunol 19:275-290, 2001).

De manière préférée, l'immunoglobuline sera de type IgG et le récepteur Fc sera le récepteur FcγRIIIa.

La CDC se réfère à la lyse des cellules cibles en présence de molécules du complément. Ce mécanisme fait référence à la voie classique du complément, la voie alterne et la voie des lectines étant indépendantes des anticorps.

Le système du complément est un ensemble de protéines sériques impliquées dans l'inflammation, l'activation de cellules phagocytaires et la lyse de membranes cellulaires. Il est constitué de différents composants tels que C1, C4, C5, C9, cette liste n'étant pas exhaustive.

Une cascade de réactions enzymatiques, impliquant une vingtaine de protéines, est déclenchée à la suite de la liaison de plusieurs C1q (un des composants de C1) à différents Fc d'anticorps liés à des antigènes présents sur la cellule cible. Les différentes réactions de protéolyse en chaîne qui en résultent, engendre une lyse par choc osmotique de la cellule cible (CDC).

De manière préférée, l'immunoglobuline sera de type IgG particulièrement IgG1 et IgG3 qui sont considérés comme efficaces alors que les IgG2 et IgG4 sont considérés comme peu actives voire inactives dans l'activation de la voie classique du complément *(*Shakib F, Basic and Clinical Aspects of IgG Subclasses, Karger, 1986).

L'ADCP est le mécanisme par lequel les anticorps (par leur fragment Fc) vont agir en tant qu'opsonines pour favoriser l'ingestion de la cellule cible par des phagocytes (cellules effectrices capable de phagocytose, neutrophiles et macrophages principalement). Les phagocytes liés à une cellule cible opsonisée l'ingèrent en l'entourant de pseudopodes. Ceux-ci fusionnent, et l'agent étranger est alors internalisé (endocyté) dans le phagocyte, désormais nommé le phagosome. Des granules et des lysosomes fusionnent avec le phagosome et versent, dans ce qui est devenu un phagolysosome, des enzymes qui digèrent ainsi la cellule cible. Les résidus seront ensuite libérés dans le milieu extracellulaire par exocytose (Munn DH et al, Cancer Research ; 51:1117-1123, 1991).

Selon un aspect, la demande concerne ainsi une composition comprenant des anticorps monoclonaux dirigés contre une cytokine pro-inflammatoire circulante, lesdits anticorps ayant une forte affinité pour le récepteur FcyRIIIa (CD16), pour son utilisation dans le cadre de la prévention ou du traitement des phases précoces de l'inflammation.

L'invention est relative à une composition comprenant des anticorps monoclonaux dirigés contre une cytokine pro-inflammatoire circulante, lesdits anticorps ayant une affinité au moins égale à $2x10^7$ $M^{-1}$ pour le récepteur FcγRIIIa (CD16) et dans laquelle le taux de fucose de l'ensemble des anticorps de ladite composition est inférieur à 60%, pour son utilisation dans le cadre de la prévention ou du traitement de l'inflammation,
notamment dans laquelle lesdits anticorps ont une affinité au moins égale à $2x10^8$ $M^{-1}$ ou $2x10^9$ $M^{-1}$, telle que déterminée par l'analyse Scatchard ou la technologie BIAcore (Label-free surface plasmon resonance based technology).

Une cytokine pro-inflammatoire circulante selon la demande ou l'invention s'entend d'une cytokine pro-inflammatoire fixée sous sa forme soluble par les anticorps de la demande ou de l'invention. La forte affinité de la région Fc de l'anticorps de la demande ou de l'invention pour le récepteur FcγRIIIa (CD16) permet à l'anticorps de la demande ou de l'invention de ne pas être déplacé par des anticorps IgG polyclonaux, notamment IgG présents dans le sérum sanguin.

Dans un autre aspect de la demande ou de l'invention, la région Fc des anticorps peut ne pas être mutée.

Par forte affinité, on désigne une affinité au moins égale à $2x10^6$ $M^{-1}$, telle que déterminée par l'analyse Scatchard ou la technologie BIAcore (Label-free surface plasmon resonance based technology)
Dans cet aspect de la demande ou de l'invention les anticorps sont uniquement dirigés contre des cytokines. Ils ne sont donc pas dirigés contre d'autres antigènes tels que l'antigène 5C5 (antigène tumoral exprimé par les cellules des carcinomes rénaux), le BCR (B Cell Receptor), un idiotype comme celui des anticorps inhibiteurs anti-FVIII, le TCR (T Cell Receptor), CD2, CD3, CD4, CD8, CD14, CD15, CD1 9, CD20, CD21, CD22, CD23, CD25, CD45, CD30, CD33, CD37, CD38, CD40, CD40L, CD46, CD52, CD54, CD66 (a, b, c, d), CD74, CD80, CD86, CD126, CD138, CD154, MUC1 (Mucine 1), MUC2 (Mucine 2), MUC3 (Mucine 3), MUC4 (Mucine 4), MUC16 (Mucine 16), HM1.24 (antigène spécifique des plasmocytes sur-exprimé dans les myélomes multiples), tenascin (protéine de la matrice extra-cellulaire), GGT (gamma- glutamyltranspeptidase), VEGF (Vascular Endothelial Growth par exemple.

Dans un mode de réalisation particulier, la composition de la demande ou de l'invention comprend des anticorps monoclonaux dirigés contre une cytokine pro-inflammatoire circulante, ayant une affinité au moins égale à $2x10^6$ $M^{-1}$, au moins égale à $2x10^7$ $M^{-1}$, $2x10^8$ $M^{-1}$ ou $2x10^9$ $M^{-1}$, telle que déterminée par l'analyse Scatchard ou la technologie BIAcore. La présente demande concerne en particulier une composition comprenant des anticorps monoclonaux dirigés contre une cytokine pro-inflammatoire circulante, le taux de fucose de l'ensemble des anticorps de ladite composition étant inférieur à 60%, et de préférence inférieur à 50%, pour son utilisation dans le cadre de la prévention ou du traitement des phases précoces de l'inflammation.

L'invention est en particulier relative à une composition comprenant des anticorps monoclonaux dirigés contre une cytokine pro-inflammatoire circulante, lesdits anticorps ayant une affinité au moins égale à $2x10^7$ $M^{-1}$ pour le récepteur

FcγRIIIa (CD16) et dans laquelle le taux de fucose de l'ensemble des anticorps de ladite composition est inférieur à 60%, pour son utilisation dans le cadre de la prévention ou du traitement de l'inflammation,
notamment dans laquelle lesdits anticorps ont une affinité au moins égale à $2x10^8 M^{-1}$ ou $2x10^9 M^{-1}$, telle que déterminée par l'analyse Scatchard ou la technologie BIAcore (Label-free surface plasmon resonance based technology).

Il était connu de l'homme du métier que le taux de fucosylation réduit de la chaine lourde des anticorps augmente leur affinité pour les récepteurs des parties constantes des anticorps (Récepteurs Fc), en particulier les récepteurs Fcγ de type III (FcγRIII) exprimés à la surface des cellules NK et des macrophages.

Alors que la littérature décrit l'impact de la forte interaction des anticorps avec le FcγRIII comme augmentant le pouvoir cytotoxique de l'anticorps contre une cellule cible et cela via l'action de cellules effectrices comme les cellules NK et les macrophages, la demande ou l'invention repose sur la constatation faite par les Inventeurs qu'une cytokine reconnue par un anticorps à forte affinité pour le CD16 sera « capturée » préférentiellement par lesdits anticorps et sera ensuite détruite par les macrophages, après phagocytose du complexe cytokine-anticorps.

Un tel mécanisme n'entre pas en compétition avec les cellules tueuses naturelles (NK), qui expriment également les FcγRIII à leur surface, et la lyse cellulaire dépendante des anticorps (ADCC) n'est pas impliquée.

Les anticorps anti-cytokine selon la demande ou l'invention servent de bio-épurateurs permettant l'élimination des cytokines.

Lesdits anticorps de la composition selon la demande ou l'invention sont tels :

- qu'ils sont dirigés contre des épitopes différents de ladite cytokine pro-inflammatoire circulante, ou
- qu'ils sont tous dirigés contre le même épitope de ladite cytokine pro-inflammatoire circulante,
- qu'ils possèdent tous les mêmes régions CDR, ou
- qu'ils possèdent tous la même séquence d'acides aminés de la chaine légère et de la chaine lourde.

Dans la demande ou l'invention, les anticorps contenus dans la composition sont soit de même nature, soit de nature différente.

Par « anticorps de même nature » on entend des anticorps possédant une séquence d'acides aminés identiques. En d'autres termes, les anticorps de même nature possèdent tous les mêmes séquences d'acides aminés de leurs chaines lourdes et de leurs chaines légères. Cependant, les anticorps de même nature selon la demande ou l'invention peuvent présenter des modifications post traductionnelles différentes : par exemple, les anticorps de même nature peuvent posséder des glycosylations différentes.

Par « anticorps de nature différente» on entend des anticorps possédant des séquences d'acides aminés différentes. Il peut s'agir soit d'anticorps présentant des différences pour toutes leurs séquences d'acides aminés, soit possédant des différences pour une partie de leurs séquences d'acides aminés, le reste des séquences étant identiques.

Ainsi, une composition d'anticorps selon la demande ou l'invention, où les anticorps sont de nature différente comprend :

- une composition d'anticorps polyclonaux,
- une composition d'anticorps présentant les mêmes régions constantes, mais où les régions variables sont différentes,
- une composition d'anticorps présentant les mêmes régions constantes, et les mêmes régions charpentes (FR) des régions variables, mais où les régions hypervariables (CDR) sont différentes, une composition d'anticorps chimères, présentant une région quelconque commune, le reste des séquences étant différentes.

La composition selon la demande ou l'invention comprend donc des anticorps, de même nature ou de nature différente, pour la prévention ou le traitement des phases précoces de l'inflammation.

La composition selon l'invention comprend donc des anticorps, de même nature ou de nature différente, pour la prévention ou le traitement de l'inflammation.

Par « phases précoces de l'inflammation », on entend dans la demande ou l'invention les premières étapes de l'inflammation, c'est-à-dire l'initiation de l'inflammation, et notamment l'étape de libération des cytokines pro-inflammatoires comme le TNF-α, IFN-γ, IFN-α, IL-1, IL-6, IL-8, IL-12, IL-17, IL-18, GM-CSF, en quantité supérieure à 1.5 fois, préférentiellement 3 fois voire supérieure à 5 fois la quantité normale.

Dans encore un autre mode de réalisation avantageux, la demande ou l'invention concerne une composition telle que définie précédemment, où chaque anticorps monoclonal compris dans ladite composition possède une affinité pour les récepteurs FcγRIII au moins 1.5 fois supérieure à celle d'un anticorps naturel dirigé contre ladite cytokine pro-inflammatoire circulante.

Dans encore un autre mode de réalisation, la demande ou l'invention concerne une composition telle que définie précédemment, où ladite cytokine pro-inflammatoire est choisie parmi les cytokines pro-inflammatoires suivantes :

- TNF-α,

- IL-1β,
- IL-6,
- IL-8,
- IL-12,
- IL-17
- IL-18
- GM-CSF

La demande ou l'invention concerne plus particulièrement une composition telle que définie précédemment, où ladite cytokine pro-inflammatoire est le TNF-α.

Avantageusement, la demande ou l'invention concerne une composition telle que définie précédemment, où chaque anticorps compris dans ladite composition ne possède pas de propriétés de neutralisation de ladite cytokine pro-inflammatoire circulante.

Les anticorps de la composition selon la demande ou l'invention sont donc capables de reconnaître un ou plusieurs épitopes de ladite cytokine pro-inflammatoire circulante. Cependant, bien que les anticorps reconnaissent spécifiquement la cytokine pro-inflammatoire, l'interaction anticorps-cytokine pro-inflammatoire ne bloque pas l'activité de la cytokine pro-inflammatoire. Ainsi, un complexe anticorps-cytokine pro-inflammatoire, s'il n'est pas éliminé par le système macrophage tel que défini dans la demande ou l'invention, serait toujours présent dans l'organisme.

Les anticorps de la demande ou de l'invention sont donc très efficaces non pas par leur propriétés neutralisantes, mais par leur capacité accrue à interagir avec les récepteurs FcγRIII, et ainsi éliminer de la circulation de l'organisme contaminé la cytokine pro-inflammatoire. La demande ou l'invention concerne également une composition telle que définie précédemment, en association avec au moins un agent anti inflammatoire.

Parmi les agents anti inflammatoire de l'invention, on peut citer les corticoïdes (glucocorticoïdes ou anti-inflammatoires stéroïdiens) et les anti-inflammatoires non-stéroïdiens. Ces agents anti inflammatoires sont bien connus de l'homme de métier.

La demande ou l'invention concerne aussi des anticorps hautement galactosylés, en particulier des anticorps anti-TNF-α, et des compositions les contenant.

Selon un autre aspect, la demande ou l'invention concerne également l'utilisation d'anticorps monoclonaux dirigés contre une toxine bactérienne circulante, pour son utilisation dans le cadre de la prévention ou du traitement des phases précoces d'une infection bactérienne liée à la libération de ladite toxine.

Les anticorps de la demande ou de l'invention fixent une toxine bactérienne circulante, sous sa forme soluble.

Par « infection bactérienne », on entend une infection provoquée par une souche bactérienne pathogène. Aux fins de la demande ou de l'invention, ladite souche bactérienne désigne toute souche de bactéries à gram positif ou toute souche de bactérie à gram négatif. La dichotomie gram positif/gram négatif est faite sur la base de la composition de la paroi des bactéries, celle des bactéries à gram positif étant très riche en peptidoglycane contrairement à celle des bactéries à gram négatif.

Les bactéries à gram positif sont par exemple celles appartenant au genre Staphylococcus, Lactobacillus, Clostridium, Enterococcus, Listeria,...

Les bactéries à gram négatif sont par exemple les bactéries de genre Salmonelle, Escherichia Coli, Pseudomonas,...

Seules les bactéries pathogènes entrainent une infection bactérienne. La pathogénicité est liée aux facteurs de virulence de la bactérie, à savoir des facteurs génétiques (chromosomiques ou extrachromosomiques), lesdits facteurs étant en effet responsables de l'implantation de la bactérie, sa multiplication ou ses effets néfastes (liés à la libération de toxines notamment). L'infection bactérienne est divisée en plusieurs phases. La première consiste en la colonisation de l'hôte par la bactérie, (par exemple lors d'une blessure profonde au moyen d'un objet contaminé par *Clostridium tetani,* par blessure ou consommation de nourriture contaminée par *Clostridium botulinum* , par les voies respiratoires par *Corynebacterium diphtheriae* ...), ladite étape étant suivie de la phase exponentielle de croissance des bactéries, phase durant laquelle lesdites bactéries luttent contre le système immunitaire et utilisent les nutriments de l'hôte dont elles ont besoin pour se développer. Vient ensuite la libération des toxines puis la liaison de ces dernières sur leurs cibles, d'où une altération du fonctionnement normal de la cellule hôte (modification des échanges électrolytiques, inhibition de synthèses protéiques...), ou bien la lyse de la cellule hôte.

Les infections bactériennes conformes à la demande ou à l'invention font référence à toutes les infections associées à la libération d'une quelconque exotoxine (telles que la toxine tétanique, la toxine botulique, la toxine diphtérique...) ou à la libération d'une quelconque endotoxine, dans la circulation sanguine.

La demande ou l'invention concerne une composition d'anticorps monoclonaux dirigés contre une toxine bactérienne circulante, présentant une affinité pour le récepteur FcγRIIIa (CD16) améliorée par rapport à des anticorps dirigés contre ladite toxine bactérienne, produits dans la lignée cellulaire CHO, pour son utilisation dans le cadre de la prévention ou du traitement des phases précoces d'une infection bactérienne liée à la libération de ladite toxine.

Les anticorps agissent ainsi pendant les phases précoces de l'infection.

Par « phases précoces d'une infection bactérienne », on entend les phases correspondantes à l'intervalle de temps s'étendant entre la libération de la toxine par la bactérie et la fixation au récepteur membranaire ou avant la pénétration de la toxine dans la cellule cible, lorsque la cible est intracellulaire. En d'autres termes, les étapes précoces de ces maladies correspondent au moment où la toxine est libre dans l'organisme et donc lorsqu'elle est accessible aux anticorps.

Cet aspect de la demande ou de l'invention repose sur la constatation faite par les Inventeurs qu'il est possible à l'aide d'anticorps possédant une forte affinité pour les récepteurs FcyRIII d'induire la clairance (l'élimination) rapide des toxines circulantes, dès leur libération par la bactérie.

Ainsi, une toxine reconnue par les anticorps conformes à la demande ou à l'invention sera « capturée » par lesdits anticorps et sera détruite par les macrophages, via la phagocytose du complexe immun formé de la toxine et de l'anticorps dirigé contre elle.

Un tel mécanisme n'entre pas en compétition avec les cellules tueuses naturelles (NK), qui expriment également les FcγRIII à leur surface, et la lyse cellulaire dépendante des anticorps (ADCC) n'est pas impliquée.

Les anticorps anti-toxine selon la demande ou l'invention servent ainsi de « bio-épurateurs » permettant l'élimination des toxines. Par « bio-épurateurs », on entend le fait que lesdits anticorps peuvent soustraire du sang les toxines en circulation.

Dans un mode de réalisation avantageux, la demande ou l'invention concerne une composition telle que définie précédemment, dans laquelle lesdits anticorps monoclonaux possèdent une affinité pour les récepteurs FcγRIIIa au moins deux fois supérieure à celle d'un anticorps produit naturellement en réponse à la présence de la toxine bactérienne dans un organisme et dirigé contre ladite toxine bactérienne, ou à celle d'anticorps produit dans la lignée cellulaire CHO.

Dans encore un autre mode de réalisation, la demande ou l'invention concerne une composition telle que définie précédemment, où ladite toxine bactérienne est choisie parmi les toxines suivantes :

- la toxine tétanique (*Clostridium tetani)*
- la toxine botulique (*Clostridium botulinum)*
- la toxine diphtérique (*Corynebacterium diphtheriae)*
- la toxine de l'Anthrax (*Bacillus anthracis*)
- la toxine pertussique *(Bordetella pertussis*),
- la toxine cholérique,
- les toxines du staphylocoque, et
- les saxitoxines.

Avantageusement, la demande ou l'invention concerne une composition telle que définie précédemment, dans laquelle, lesdits anticorps ne possèdent pas nécessairement de propriétés de neutralisation de ladite toxine circulante. En effet, lesdits anticorps sont capables de reconnaître un ou plusieurs épitopes de la toxine circulante, selon qu'ils sont monoclonaux ou polyclonaux et bien qu'ils reconnaissent spécifiquement la toxine, ils ne bloquent pas son activité. Les anticorps de la demande ou de l'invention sont donc très efficaces non pas par leur propriétés neutralisantes, mais par leur capacité accrue à interagir avec les récepteurs FcyRIII, et ainsi éliminer la toxine de la circulation de l'organisme contaminé.

Dans un autre mode de réalisation avantageux, la demande ou l'invention concerne une composition susmentionnée, où l'infection bactérienne liée à la libération de ladite toxine est choisie parmi : le tétanos, le botulisme, la diphtérie, la coqueluche, la maladie du charbon, le choléra, les infections aux staphylocoques, les intoxications dues aux saxitoxines.

L'homme de métier sait que la toxine tétanique est responsable du tétanos, la toxine botulique est responsable du botulisme, la toxine diphtérique est responsable de la diphtérie, la toxine pertussique est responsable de coqueluche, la toxine de l'Anthrax est responsable de la maladie du charbon, la toxine cholérique responsable du choléra, peut entrainer des oedèmes pulmonaires engendrant des syndromes de détresse respiratoire aigüe, la toxine staphylococcique est responsable notamment d'intoxications alimentaires, les saxitoxines sont responsables de déficiences respiratoires et/ou paralysies.

La composition d'anticorps selon la demande ou l'invention peut également être couplée à un traitement antibiotique afin d'éliminer rapidement de la circulation, les toxines libérées lors de la lyse bactérienne engendrée par le traitement antibiotique. La composition d'anticorps selon la demande ou l'invention est tout particulièrement conseillée dans le cadre d'un traitement antibiotique visant une infection à bactérie à gram négatif qui en se lisant, vont libérer de manière massive les endotoxines contenues dans leurs parois.

La demande ou l'invention concerne également une composition telle que définie précédemment, en association avec au moins un anticorps dirigé contre au moins un épitope d'une protéine exprimée à la surface de la bactérie produisant ladite toxine bactérienne circulante.

Ainsi, la composition selon la demande ou l'invention comprend :

- des anticorps anti toxine, tels que définis précédemment, capables de capturer une toxine circulante, et de l'éliminer par phagocytose, et
- au moins un anticorps capable de reconnaître au moins un épitope d'une protéine exprimée à la surface d'une bactérie exprimant ladite toxine, ce second anticorps permettant la stimulation du système immunitaire et la lyse des bactéries.

En effet, certaines bactéries sont pathogènes par la libération de leur toxine mais également pas leur simple présence dans l'organisme. En conséquence, cette composition est très avantageuse car elle permet non seulement d'éliminer la toxine, nocive pour l'organisme, mais également d'empêcher la multiplication, ou la survie de la bactérie produisant ladite toxine.

L'anticorps dirigé contre au moins un épitope d'une protéine exprimée à la surface de la bactérie produisant la toxine est de préférence un anticorps présentant une capacité augmentée à lyser la bactérie. En d'autres termes, ledit anticorps présente une forte capacité à induire de l'ADCC.

Alternativement, la demande ou l'invention concerne une composition telle que définie précédemment, en association avec au moins un anticorps neutralisant dirigé contre ladite toxine bactérienne circulante.

Encore plus avantageusement, la demande ou l'invention concerne une composition précédemment décrite, en association :

- avec au moins un anticorps dirigé contre au moins un épitope d'une protéine exprimée à la surface de la bactérie produisant ladite toxine bactérienne circulante, et
- au moins un anticorps neutralisant dirigé contre ladite toxine bactérienne circulante.

La composition susmentionnée comprend donc trois types d'anticorps :

- des anticorps anti toxine, tels que définis précédemment, capables de capturer la toxine circulante, et de l'éliminer par phagocytose,
- au moins un anticorps neutralisant, dont l'effet est d'empêcher la toxine circulante d'exercer son effet nocif, sans pour autant permettre l'élimination de la toxine circulante, et
- au moins un anticorps capable de reconnaître au moins un épitope d'une protéine exprimée à la surface d'une bactérie exprimant ladite toxine, ce second anticorps permettant la stimulation du système immunitaire et la lyse des bactéries.

Cette composition est très avantageuse car elle permet de neutraliser la toxine, l'éliminer par phagocytose et éliminer la bactérie produisant ladite toxine.

Dans un autre mode de réalisation avantageux, la demande ou l'invention concerne une composition telle que définie précédemment, où lesdits anticorps et

- ledit antibiotique, et/ou
- ledit anticorps dirigé contre au moins un épitope d'une protéine exprimée à la surface de la bactérie produisant ladite toxine bactérienne circulante, et/ou
- ledit anticorps neutralisant dirigé contre ladite toxine bactérienne circulante sont utilisés de manière simultanée, séquentielle ou séparée dans le temps.

Dans encore un autre mode de réalisation avantageux, la demande ou l'invention concerne une composition définie précédemment, c'est-à-dire comprenant des anticorps dirigés soit contre une cytokine pro-inflammatoire circulante, soit contre une toxine bactérienne circulante, où ladite composition est utilisée à des doses variant de environ 0,05mg/m$^2$ à 2000mg/m$^2$, en particulier de 10mg/m$^2$ à environ 2000mg/m$^2$, en particulier, la dose unitaire administrée peut variée de 15mg à environ 3g par patient.

La dose unitaire administrée peut varier de 100$\mu$g à environ 1g par patient.

Encore plus avantageusement, la demande ou l'invention concerne une composition telle que définie précédemment, ladite composition étant sous forme injectable, ou sous forme de spray.

La composition de la demande ou de l'invention est administrée notamment par voie intraveineuse, lorsqu'elle se présente sous forme d'un liquide injectable. Elle peut être administrée par les voies respiratoires lorsqu'elle se présente sous forme de spray.

La composition de la demande ou de l'invention peut être administrée notamment par voie intraveineuse, par voie sous-cutanée, par voie systémique, par voie locale au moyen d'infiltrations ou per os.

Le traitement peut être continu ou séquentiel, c'est-à-dire au moyen d'une perfusion délivrant ladite composition de manière continue et éventuellement constante, ou sous forme discontinue, par une ou plusieurs prises ou injections quotidiennes, éventuellement renouvelées plusieurs jours, soit consécutifs, soit avec une latence sans traitement entre les prises.

L'administration de ladite composition peut être continue au moyen d'une perfusion intraveineuse, délivrant ladite composition soit à débit constant soit à débit variable sur quelques heures à plusieurs jours. L'administration de ladite composition peut également se faire de façon discontinue ou séquentielle, par une ou plusieurs prises ou injections quotidiennes, éventuellement renouvelées sur plusieurs jours.

Dans un autre mode de réalisation avantageux, l'invention concerne une composition telle que définie précédemment en association avec un véhicule pharmaceutiquement acceptable.

Les anticorps conformes à la demande ou l'invention, qu'ils soient dirigés contre une cytokine pro-inflammatoire circulante ou qu'ils soient dirigés contre une toxine bactérienne peuvent être produits par diverses techniques connues de l'homme du métier, notamment celles décrites ci-après.

Les anticorps chimériques selon la demande ou l'invention peuvent être préparés en utilisant les techniques de recombinaison génétique. Par exemple, un anticorps chimérique peut être réalisé en construisant un gène chimère comprenant une séquence codant pour la région variable de la chaîne lourde d'un anticorps monoclonal murin, liée grâce à un linker à une séquence codant pour la région constante de la chaîne lourde d'un anticorps humain, et en construisant un gène chimère comprenant une séquence codant pour la région variable de la chaîne légère d'un anticorps monoclonal murin, liée grâce à un linker à une séquence codant pour la région constante de la chaîne légère d'un anticorps humain. En transfectant lesdits gènes chimères, par fusion de protoplastes ou toute autre technique, dans une lignée cellulaire, de myélome murin par exemple, on obtient la production d'anticorps chimériques souris-homme par les cellules transformées. C'est le document Morrison et al., Proc. Natl. Acad. Sci. U.S.A., 81, pp. 6851-55 (1984) qui a décrit pour la première fois la préparation de tels anticorps. Les documents Verhoeven et al., BioEssays, 8:74, 1988, Boulianne, G. L. et al., Nature, 312:643 (1984), Sun, L.K., et al., Proc. Natl. Acad. Sci. USA 84, 214-218, US 4,816,567, US 6,331,415, US 6,808,901 et EP 125023 pourront également être utilisés comme référence par l'homme du métier, de même que Bobrzecka, K., et al, Immunology Letters 2, pp 151-155 qui décrit une procédure de fractionnement des ponts disulfures interchaînes des immunoglobulines suivie par un réarrangement ordonné de ces mêmes ponts disulfures afin d'obtenir des anticorps formés de fragments Fab de lapin et de fragments Fc humain.

Une autre approche de préparation d'anticorps chimériques, telle que décrite dans le document FR 2 641 468, peut être de greffer des fragments Fab' d'un anticorps monoclonal murin sur des immunoglobulines polyclonales humaine, notamment IgG, ou sur des fragments Fc, ceci à l'aide d'un agent de couplage, par exemple un diimide. Des anticorps chimériques de type Ig-Fab' (également noté Fab'-Ig), Fc-Fab' ou (Fab')$^2$ peuvent ainsi être obtenus. De tels anticorps chimériques sont caractérisés par la greffe de l'ensemble du fragment Fab', et non seulement des parties variables.

Alternativement, d'autres auteurs ont décrit l'obtention d'anticorps chimériques monovalents par greffage de fragments Fab' d'anticorps polyclonaux sur des IgG ou sur des fragments Fc (G.T. Stevenson et al, Med. Oncol. & Tumor, 1985, Pharmacother, vol. 1, n°4, 275-278, 1984).

La recombinaison homologue *in vivo* entre les portions des gènes codant pour les régions constantes des chaînes légères et des chaînes lourdes d'une immunoglobuline murine par des portions des gènes codant pour les régions constantes des chaînes légères et des chaînes lourdes d'une immunoglobuline humaine est également un moyen qui peut être utilisé afin d'obtenir de tels anticorps (US 5,204,244 ou US 5,202,238).

Cette liste n'est pas exhaustive.

Les anticorps humanisés selon la demande ou l'invention peuvent également être préparés par des techniques bien connues, telles que celle décrites pour la première fois dans le document Jones et al., Nature, 1986, 321-522-525. Il s'agit du remplacement des régions hypervariables (CDRs) d'un anticorps humain par des régions hypervariables d'origine murine, à la fois au niveau des chaînes légères mais aussi des chaînes lourdes. Cette technique, aujourd'hui bien connue de l'homme de l'art sous le nom de *«CDR grafting»* a été décrite dans de nombreux documents tels que Singer et al., J. Immun. 150:2844-2857 (1993), Riechman et al., Nature 323:326 (1988), Verhoeyen et al., Science 239:1534 (1988) ou encore les brevets US 5,225,539 ; US 5,585,089 ; EP 0682040 qui pourront également être utilisés comme référence.

La plupart des anticorps humanisés réalisés par greffage des régions CDRs présentent néanmoins une affinité réduite par rapport à un anticorps murin, ceci en raison du rôle majeur de certains acides aminés des régions charpentes, les régions adjacentes aux régions CDRs. C'est pourquoi aujourd'hui l'homme du métier remplace, bien souvent, non seulement les CDRs, mais aussi les résidus des régions charpentes susceptibles de contribuer au site de liaison de l'antigène (Studicka *et al.,* 1994).

Une autre technique qui permet d'humaniser des anticorps est la technique de greffage des résidus déterminants spécifiques (*Specificity Determining Region, SDR*), qui consiste à greffer non plus l'ensemble des régions CDRs, mais uniquement les régions SDRs de l'anticorps non-humain dans les régions variables humaines (Tamura et al., J Immunol. 2000; 164 : 1432-41). Les régions SDRs sont définies comme les régions des CDRs en contact direct avec l'antigène

(Padlan et al. (1995), FASEB J. 9: 133-139). Cette technique nécessite donc l'identification des SDRs. Cela peut se faire, par exemple, par une détermination de la structure 3D du complexe antigène-anticorps, en utilisant la base de données des SDRs déjà identifiés (http://paradox.harvard.edu/sdr), ou bien grâce à des comparaisons des séquences variables humaines à celles de l'espèce non-humaine, ceci à l'aide de logiciels informatiques tels que CLUSTALW2, CLUSTALX, BLAST ou FASTA.

Une autre alternative pour obtenir des anticorps humanisés consiste à greffer des régions dites « CDRs abrégés » (*Grafting of abbreviated CDRs*). Il s'agit de la greffe des régions SDRs et de quelques résidus adjacents, en amont et en aval de la séquence. Les documents De Pascalis et al., The Journal of Immunology, 2002, 169: 3076-3084 ; Kashmiri Syed V.S et al., Humanized Antibodies and their Applications, Volume 36, Issue , May 2005, Pages 25-34 pourront être utilisés comme référence.

La technique du « *variable domaine resurfacing* », aussi nommée « *veneering* » telle que développée par ImmunoGen (US 5,639,641) peut également être utilisée. Cette technologie consiste à donner un « profil » humain à un domaine variable de souris en remplaçant les résidus exposés à la surface dans les régions charpentes des anticorps murins par les résidus habituellement trouvés à la surface des anticorps humains. Les documents Roguska et al., Proc Natl Acad Sci USA 1994 ; Mark G. E. et al (1994) in Handbook of Experimental Pharmacology vol. 113: The pharmacology of monoclonal Antibodies, Springer-Verlag, pp 105-134 pourront également servir de référence.

La plateforme Germliner™ développée par AvantGen peut également être utilisée (http://www.avantgen.com/AvantGensTechnologiesandServices.pdf). Cela permet d'obtenir des anticorps humanisés dans lesquels seuls des CDR3 sont d'origine non-humaine.

Cette liste n'est pas exhaustive.

L'obtention des anticorps selon la demande ou l'invention est, de plus, préférentiellement couplée à un processus de maturation d'affinité.

Divers techniques de mutation, aléatoire ou dirigée, bien connues dans l'état de la technique, sont utilisées afin d'augmenter l'affinité desdits anticorps. Ces dernières miment *in vitro* le processus de maturation d'affinité. Les anticorps ainsi produits sont ensuite sélectionnés, notamment sur *phage display.*

L'introduction de mutations par échange de chaînes (*chain shuffling*), telle que décrite dans les documents Clakson *et al.,* (1991) ; Marks *et al.,* (1992) ; S.G. Park *et al* (2000) est une des premières techniques qui peut être utilisée. Des domaines VL et VH de forte affinité sont sélectionnés indépendamment dans différentes librairies de domaines, puis fusionnés. Une sélection permet ensuite d'isoler le clone de plus forte affinité, ladite affinité étant de l'ordre de celle d'un anticorps isolé lors d'une réponse secondaire *in vivo.* Lorsque ce n'est pas l'intégralité du domaine qui est muté, on parle de *DNA shuffling* (Crameri et al., Nature medicine, 2(1):100-2 (1996).

Une technique alternative peut être la PCR à fort taux d'erreur (*error-prone PCR*), décrite par Hawkins *et al.,* 1992 ; Gram *et al.,* 1992. Cette PCR est caractérisée par l'emploi d'une polymérase qui induit beaucoup plus d'erreur qu'une enzyme classique ne le fait lors d'une amplification par PCR (en moyenne 1,7 base changée par domaine variable). De nombreuses mutations aléatoires sont ainsi insérées dans les séquences. La banque obtenue est ensuite sélectionnée à l'aide de l'antigène afin de sélectionner les clones d'affinités accrues.

La dégénérescence par mutagénèse dirigée permet également d'augmenter l'affinité des anticorps. Il s'agit de mutations au niveau des acides aminés situés dans les boucles hypervariables. Cette technique peut être appliquée à tous les CDRs, les uns après les autres, et les gains d'affinité peuvent être additifs (Barche *et al.,* 1994 ; Balint *et al.,* 1993). Une variante de cette technique dite le *CDR walking* consiste à ne muter les anticorps qu'au niveau de six CDRs maximum (Yang et al., J. Mol. Biol. 254, 392-403 (1995). Le document FR 2 924 431 décrit l'obtention d'anticorps de haute affinité par ladite technique. L'utilisation de souches mutagènes préalablement sélectionnées pour leur capacité à induire un taux élevé de mutations somatiques dans les domaines variables d'immunoglobulines est également un moyen d'obtenir des anticorps avec une forte affinité. Le document Holliger *et al.,* (1995) en est un exemple. Il décrit l'utilisation de la souche mutD5 qui induit des mutations ponctuelles, notamment le long du gène du scFv. Les anticorps ainsi produits sont alors sélectionnés contre l'antigène, la stringence des sélections étant augmentée à chaque tour de sélection. Le document S.J. Cumbers, et al., Nature Biotechnology, 20, 1129-1134 (2002) pourra également être pris comme référence.

La technique du *phage display* utilisant des vecteurs modifiés tels que décrits dans le document WO 2007/074496 ou la sélection sur *phage display* suivie de celle de *Biopanning* (Krebber *et al.,* (1997) ; WO 2006/117699) est également une autre alternative à l'obtention d'anticorps de haute affinité.

Lesdites technologies peuvent également, aux fins de la demande ou de l'invention, être utilisées avec des méthodes dérivées du *phage display* telles que le *ribosome display* (Irving., R.A *et al.,* (2001)) ou le *yeast display (*Chao, G et al., J. Mol. Biol. 342(2):539-50, 2004). Une autre alternative bien connue de l'homme du métier est encore d'utiliser la technique d'évolution moléculaire MutaGen™, développée par Millegen, et décrite dans le brevet US 7,670,809. Cette technologie permet de mimer l'hypermutation somatique, un phénomène naturel observé lors de la maturation des anticorps *in vivo.*

La technologie Massive Mutagenesis®, développée par Biomethodes (EP 1311670) est également une solution

possible. Il s'agit d'une technique intermédiaire entre la mutagénèse dirigée et la mutagénèse aléatoire. La production d'anticorps de haute affinité obtenue par ladite technologie est décrite dans le document WO 2009/050388.

Ces différentes technologies ne sont pas exhaustives.

Les anticorps dirigés soit contre une cytokine pro-inflammatoire circulante, soit contre une toxine bactérienne circulante contenus dans la composition selon la demande ou l'invention présentent une haute affinité pour les FcγRIII. En particulier, cette haute affinité peut être associée à une faible quantité de fucose sur les chaines glycanniques portées par les anticorps. Cette quantité de fucose, ou taux de fucose, est défini comme la proportion moyenne de fucose portée par tous les anticorps, par rapport à la quantité maximale de fucose que peuvent porter les chaines glycanniques.

Le taux de fucose peut être défini de deux manières :

- soit l'on considère que tous les anticorps de la composition sont fucosylés de la même manière, et l'on raisonnera en termes de fucosylation pour un anticorps, ledit anticorps étant représentatif de la composition,
- soit l'on considère que chaque anticorps est fucosylé de manière différente, et le taux de fucose sera la moyenne de la fucosylation individuelle de chaque anticorps composant la composition de la demande ou de l'invention.

Dans le premier cas susmentionné, si un anticorps comporte 1 site de N-glycosylation par chaine lourde, et que chaque site de glycosylation est susceptible de fixer une chaine glycannique portant un fucose, ledit anticorps aura donc la possibilité de comporter au maximum 2 fucoses. Ainsi, la population d'anticorps comportant en moyenne 1 fucose aura alors un taux de fucose de 50% (soit 1x100/2).

Dans le second cas susmentionné, si la composition est composée de 10 anticorps, par exemple 3 anticorps ne sont pas fucosylés, 3 anticorps portent un fucose et 4 anticorps portent 2 fucoses (chaque anticorps pouvant contenir jusqu'à 2 fucoses), le taux de fucosylation de la composition sera de 55%, soit 11 fucoses sur 20 possibles.

Les anticorps de la composition selon la demande ou l'invention peuvent de préférence être produits par des clones dérivés des lignées cellulaires telles que Vero (ATCC n° CCL 81), YB2/0 (ATCC n° CRL 1662) ou CHO Lec-1 (ATCC n° CRL 1735).

Dans un mode de réalisation avantageux, la demande ou l'invention concerne une composition telle que définie précédemment, où chacun des anticorps dirigés soit contre une cytokine pro-inflammatoire circulante, soit contre une toxine bactérienne circulante possède sur le site de glycosylation en position 297 de ses chaines lourdes, l'une des formes glycanniques biantennées choisie parmi les structures suivantes :

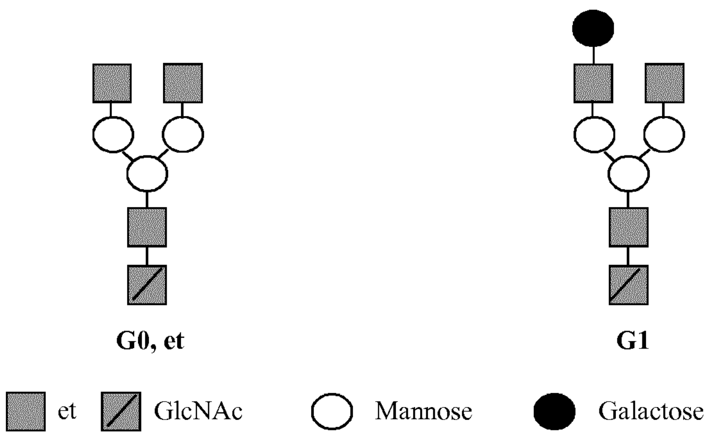


le GlcNAc représenté par 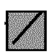 dans les structures G0 et G1 ci-dessus étant susceptible d'être fucosylé.

Les anticorps de la composition selon la demande ou l'invention portent sur l'acide aminé en position 297 de chacune des chaines lourdes une structure glycannique particulière conférant une activité effectrice dépendante du FcγRIII.

De tels anticorps peuvent être obtenus à partir d'un procédé connu de l'homme du métier, tel que décrit dans WO 01/77181, et possèdent sur leur site de glycosylation (Asn 297) des structures glycanniques de type biantennées, avec des chaînes courtes et une faible sialylation. De préférence, leur structure glycannique présente des mannoses terminaux et/ou des N- acétyl-glucosamine (GlcNAc) terminaux non intercalaires.

Le GlcNAc représenté par 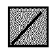 dans les structures G0 et G1 peut donc être soit non fucosylé, soit porter une molécule de fucose.

Dans un autre mode de réalisation avantageux, la demande ou l'invention concerne une composition définie

ci-dessus, où chacun des anticorps possède sur le site de glycosylation en position 297 de ses chaines lourdes, l'une des formes glycanniques biantennées choisie parmi les structures suivantes :

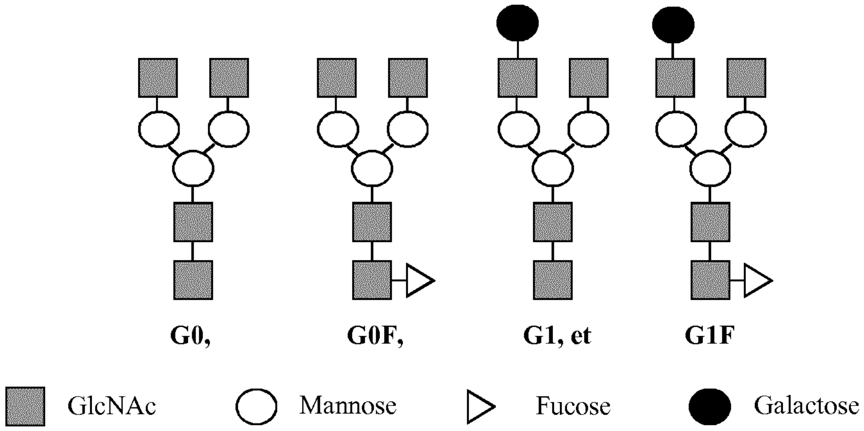


Ainsi, les anticorps contenus dans la composition de la demande ou de l'invention sont caractérisés en ce qu'ils possèdent sur son site de glycosylation Asn 297 :

- des structures glycanniques de type biantennées, avec des chaînes courtes,
- une faible sialylation,
- des mannoses et/ou des GIcNAc terminaux non intercalaires.

De manière avantageuse, les anticorps compris dans la composition selon la demande ou l'invention ont une teneur moyenne en acide sialique inférieure à 25%, 20%, 15%, ou 10%, de préférence 5%, 4% 3% ou 2%. Le taux de sialylation est défini de la même manière que le taux de fucose, comme précisé ci-dessus.

Dans encore un autre mode de réalisation avantageux, la demande ou l'invention concerne une composition décrite précédemment, caractérisée en ce que les formes G0F + G1F des anticorps de ladite composition représentent moins de 50% des structures glycanniques portées par le site de glycosylation en position 297 de la chaîne lourde (Asn 297).

Ainsi, moins de la moitié des anticorps contenus dans la composition selon la demande ou l'invention possèdent en position 297 une chaine glycannique biantennée portant un fucose. De manière encore plus avantageuse, le taux de fucose est d'environ 20% à environ 45%.

Un autre mode de réalisation avantageux de la demande ou de l'invention concerne une composition mentionnée précédemment, où les formes G0 + G1 + G0F + G1F des anticorps de ladite composition représentent plus de 60% desdites structures glycaniques et de préférence plus de 80% des structures glycanniques portées par le site de glycosylation en position 297 de la chaîne lourde.

La demande ou l'invention concerne également des populations d'anticorps dirigés soit contre une cytokine pro-inflammatoire circulante, soit contre une toxine bactérienne circulante lesdits anticorps ayant un taux élevé de galactosylation, et les compositions les contenant.

La demande ou l'invention concerne aussi un procédé de production desdits anticorps dirigés soit contre une cytokine pro-inflammatoire circulante, soit contre une toxine bactérienne circulante, lesdits anticorps ayant un taux élevé de galactosylation.

La demande ou l'invention concerne aussi un anticorps dirigé soit contre une cytokine pro-inflammatoire circulante, soit contre une toxine bactérienne circulante, l'anticorps étant hautement galactosylé.

La demande ou l'invention concerne une composition définie ci-dessus, dans laquelle le taux de galactosylation de l'ensemble des anticorps de la population est d'au moins 60%.

La demande ou l'invention concerne une composition définie ci-dessus, dans laquelle le taux de galactosylation de l'ensemble des anticorps de la population est d'au moins 70%.

La demande ou l'invention concerne une composition définie ci-dessus, dans laquelle le taux de galactosylation de l'ensemble des anticorps de la population est d'au moins 80%.

La demande ou l'invention concerne une composition définie ci-dessus, dans laquelle le taux de fucosylation de l'ensemble des anticorps de la population est d'au moins 50%.

La demande ou l'invention concerne une composition définie ci-dessus, dans laquelle le taux de fucosylation de l'ensemble des anticorps de la population est d'au moins 60%.

La demande ou l'invention concerne une composition définie ci-dessus, dans laquelle la population comprend

des anticorps qui comprennent des N-glycanes mono-galactosylés.

La demande ou l'invention concerne une composition définie ci-dessus, dans laquelle la population comprend des anticorps qui comprennent des N-glycanes bi-galactosylés.

La demande ou l'invention concerne une composition définie ci-dessus, dans laquelle le rapport du taux de galactosylation des anticorps de la population et du taux de fucosylation des anticorps de la population est compris de 1,0 à 1,4.

La demande ou l'invention concerne une composition définie ci-dessus, dans laquelle au moins 35% des anticorps dans la population comprennent des N-glycanes bi-galactosylés et au moins 25% des anticorps dans la population comprennent des N-glycanes mono-galactosylés.

La demande ou l'invention concerne une composition définie ci-dessus, dans laquelle l'anticorps est produit dans les cellules épithéliales mammaires d'un mammifère non-humain. La demande ou l'invention concerne une composition définie ci-dessus, dans laquelle l'anticorps est produit dans un mammifère non-humain transgénique, en particulier dans une chèvre, un mouton, un bison, un chameau, une vache, un cochon, un lapin, un buffle, un cheval, un rat, une souris ou un lama.

La demande ou l'invention concerne une composition définie ci-dessus, comprenant également du lait.

La demande ou l'invention concerne une composition définie ci-dessus, comprenant également un véhicule pharmaceutiquement acceptable.

La demande ou l'invention concerne également des populations d'anticorps avec un taux élevé de mannosylation, et les compositions les contenant.

La demande ou l'invention concerne aussi un procédé de production desdits anticorps avec un taux élevé de mannosylation.

La demande ou l'invention concerne aussi un anticorps, l'anticorps étant hautement mannosylé.

*<u>Populations d'anticorps</u> dirigés soit contre une cytokine pro-inflammatoire circulante, soit contre une toxine bactérienne circulante*

Les anticorps conformes à la demande ou l'invention portent une chaine oligosaccharidique au niveau de chaque asparagine 297 (Asn297, numérotation Kabat) de chacune des chaines lourdes.

Il est bien connu de l'homme du métier que des anticorps possédant des chaînes oligosaccharidiques contenant peu ou pas de fucose, présentent une meilleure affinité pour le CD16 (Fc$\gamma$RIIIa) présent sur les cellules effectrices du système immunitaire.

Ainsi, une composition d'anticorps ayant une teneur en fucose inférieure à 65% au niveau des chaines oligosaccharidiques portées par Asn297 est particulièrement préférée. La teneur en fucose peut être mesurée par des méthodes bien connues, par exemple par la méthode MALDI-TOF, HPCE-LIF, HPLC.

Avantageusement, la demande ou l'invention concerne une composition telle que définie ci-dessus dans laquelle le taux de fucose des anticorps, ou la teneur en fucose des anticorps, est inférieure à 60%, ou inférieur à 50%, ou inférieure 40%, ou au moins 30%, ou même égale à 0%. Etant entendu qu'une teneur en fucose égale à 0% correspond à 100% des chaines oligosaccharidiques portées par Asn297 qui sont sans fucose. Alternativement, la teneur en fucose peut être comprise entre 0% et 50% ou entre 10% et 50% ou entre 20 et 50%.

Dans un mode de réalisation particulier, les anticorps conformes à la demande ou l'invention peuvent comprendre en outre des chaines oligosaccharidiques qui présentent une bissection. On entend par « bissection », au sens de la demande ou de l'invention, tout résidu *N*-acétylglucosamine intercalaire greffé en $\beta$1,4 (GlcNac intercalaire), notamment par action de la $\beta$1,4-N-Acetylglucosaminyltransferase III (GnTIII).

Dans un mode de réalisation particulier, une composition d'anticorps conformes à la demande oul'invention présente une teneur en bissection d'au moins 20%, par exemple au moins 30%, ou au moins 40%, ou encore au moins 50%, 60%, 70%.

Des méthodes pour produire des anticorps conformes à la demande ou l'invention comprenant au moins un domaine Fc d'une immunoglobuline et ayant des résidus N-acétylglycosamine intercalaires (GlcNac intercalaire) sont par exemple décrites dans les documents EP 1 071 700 et US 6 602 684 ou EP 1 692 182 et US 2005/123546, cette liste n'étant pas limitative. Par exemple, les anticorps peuvent être produits dans une cellule hôte exprimant au moins un acide nucléique codant pour un polypeptide ayant une activité de $\beta$-(1,4)-N-acétylglucosaminyltransférase III en une quantité suffisante pour modifier la glycosylation portée par le(s) domaine(s) Fc dudit anticorps.

Dans un autre mode de réalisation, les anticorps présentent une faible fucosylation, c'est-à-dire des structures glycanniques ayant une teneur en fucose inférieure à 65%. Dans un mode de réalisation particulier, les anticorps présentent des structures glycanniques ayant une teneur inférieure à 50% pour les formes G0F+G1F. Dans un mode de réalisation particulier, les anticorps comprennent une teneur supérieure à 60% pour les formes G0+G1+G0F+G1F, les formes G0F+G1F étant inférieures à 50%. Dans un autre mode de réalisation particulier, les anticorps comprennent une teneur supérieure à 60% pour les formes G0+G1+G0F+G1F, la teneur en fucose étant inférieures à 65%. Ces

formes sont sélectionnées parmi les formes G0, G0F, G1 et G1F telles que décrites dans la présente demande.

Dans cet autre mode de réalisation, les anticorps comprennent en outre, sur les sites de glycosylation Asn297, une structure glycannique présentant des mannoses terminaux et/ou des N-acétylglucosamines terminaux non intercalaires.

Dans un mode de réalisation particulier, les anticorps comprennent, sur le site de glycosylation Asn297, une structure glycannique de type biantennée, avec des chaînes courtes, une faible sialylation, et une teneur supérieure à 60% pour les formes G0+G1+G0F+G1F, les formes G0F+G1F étant inférieures à 50%.

En particulier, les anticorps possèdent une teneur en acide sialique inférieure à 25%, 20%, 15%, ou 10%, de préférence 5%, 4% 3% ou 2%.

Dans un mode de réalisation particulier, les anticorps présentent des structures glycanniques telles que décrites dans WO 01/77181. Les anticorps peuvent notamment être sélectionnés en sélectionnant une lignée cellulaire capable de produire des anticorps présentant une forte affinité pour le récepteur CD16. Par exemple, les anticorps peuvent être produits dans un hybridome, notamment un hétérohybridome obtenu avec le partenaire de fusion K6H6-B5 (ATCC CRL 1823) ou dans une cellule animale ou humaine produisant ledit anticorps, notamment une cellule dérivée des lignées Vero (ATCC CCL-81), une lignée cellulaire d'hybridome de rat, comme par exemple la lignée d'hybridome de rat YB2/0 (ATCC CRL-1662, cellule YB2/3HL.P2.G11.16Ag.20, déposée à l'American Type Culture Collection) ou encore la lignée CHO Lec-1 (ATCC CRL-1735), CHO-Lec10, CHO dhfr- (par exemple CHO DX BI1, CHO DG44), CHO Lec13 , SP2/0, NSO, 293, BHK, COS, IR983F, un myélome humain comme Namalwa ou toute autre cellule d'origine humaine comme PERC6, CHO Pro-5, CHO dhfr- (CHO DX BI1, CHO DG44), Wil-2, Jurkat, Vero, Molt-4, COS-7, 293- HEK, BHK, K6H6, NSO, SP2/0-Ag 14 et P3X63Ag8.653.

Avantageusement, les anticorps peuvent être produits dans une lignée cellulaire choisie parmi YB2/0, Vero, CHO-lec10, CHO-lec13, CHO-lecl, CHOK1SV, CHO Knock Out pour la fucosyltransférase FUT8, CHO exprimant la GnTIII.

De manière particulièrement avantageuse, les anticorps sont produits dans une lignée cellulaire d'hybridome de rat. Dans un mode de réalisation préféré, les anticorps sont produits dans l'hybridome de rat YB2/0 (cellule YB2/3HL.P2.G11.16Ag.20, déposée à l'American Type Culture Collection sous le numéro ATCC CRL-1662) sélectionnée pour sa capacité à produire des anticorps présentant une forte affinité pour le CD16.

D'autres méthodes sont connues de l'homme du métier pour produire des anticorps à glycosylation optimisée. Par exemple l'utilisation d'inhibiteur de la glycosylation comme la kifunensine (inhibiteur de l'alpha mannosidase II), qui peut être ajoutée au milieu de culture des cellules productrices d'anticorps, selon la méthode décrite dans US 7 700 321. Des analogues de fucose peuvent également être introduits dans le milieu de culture de cellules productrices d'anticorps comme décrit dans le document US 20090317869. Un autre moyen de produire des anticorps à glycosylation optimisée, est l'utilisation de cellules pour lesquelles la voie de production des GDP-fucose est inhibée, par le biais de l'inhibition de l'une au moins des enzymes du cycle de production du fucose, telle que décrit par exemple dans le document US 2010291628 ou US 20090228994, le document EP 1 500 698, le document EP 1 792 987 ou encore le document US 7 846 725, cette liste n'étant pas limitative. Il est également possible d'utiliser des ARN interférence (ARNi) inhibant la 1,6-fucosyltransférase comme décrit dans le document US 7 393 683 ou le document WO 2006133148.

D'autres méthodes de préparation d'anticorps à glycosylation optimisée dans des animaux transgéniques sont décrites dans WO 200748077. Les anticorps peuvent également être produits dans des levures, comme cela est montré dans le document WO 0200879.

Pour produire des anticorps avec 100% d'oligosaccharides non fucosylés, c'est-à-dire totalement dépourvus de fucose au niveau de la glycosylation portée par l'Asn297, il est possible de mettre en oeuvre les méthodes de préparation décrites dans les documents EP 1 176 195, US 7 214 775, US 6 994 292, US 7 425 446, US2010223686, WO2007099988, EP 1 705 251, cette liste n'étant pas limitative. Dans un mode de réalisation particulier, les anticorps sont produits dans des cellules modifiées par délétion du gène codant pour l'$\alpha$1,6- fucosyltransférase ou par addition d'une mutation de ce gène pour éliminer l'activité $\alpha$1,6- fucosyltransférase.

Selon un autre aspect, la demande ou l'invention concerne une composition comprenant une population d'anticorps dirigés soit contre une cytokine pro-inflammatoire circulante, soit contre une toxine bactérienne circulante, et dans lequel le taux de galactosylation des anticorps de la population est d'au moins 60%.

Selon encore un autre aspect, le taux de galactosylation des anticorps de la population est d'au moins 70%.

Selon encore un autre aspect, le taux de galactosylation des anticorps de la population est d'au moins 80%.

Selon encore un autre aspect particulier, le taux de fucosylation de l'ensemble des anticorps de la population est d'au moins 50%, et notamment d'au moins 60%.

Selon un autre aspect particulier, la population comprend des anticorps qui comprennent des N-glycanes monogalactosylés.

Selon un autre aspect particulier, la population comprend des anticorps qui comprennent des N-glycanes bigalactosylés.

Selon un autre aspect particulier, le rapport du taux de galactosylation des anticorps de la population et du

taux de fucosylation des anticorps de la population est compris de 1,0 à 1,4. Selon un autre aspect particulier, au moins 35% des anticorps dans la population comprend des N-glycanes bi-galactosylés et au moins 25% des anticorps dans la population comprend des N-glycanes mono-galactosylés.

Selon un autre aspect particulier, le taux de sialylation des anticorps est d'au moins 50%. Selon encore un autre aspect particulier, le taux de sialylation des anticorps est d'au moins 70%.

Selon encore un autre aspect particulier, le taux de sialylation des anticorps est d'au moins 90%.

Selon encore un autre aspect particulier, les anticorps sont totalement sialylés.

La biosynthèse des N-glycanes n'est pas régulée par une codification, comme cela est le cas avec les protéines, mais est principalement dépendante de l'expression et de l'activité des glycosyltransférases spécifiques dans une cellule. Ainsi, une glycoprotéine, tel le fragment Fc d'un anticorps, existe normalement comme une population hétérogène de glycoformes qui portent différents glycannes sur le même squelette de protéines.

Une population d'anticorps hautement galactosylés, est une population d'anticorps dans laquelle le taux de galactosylation de l'ensemble des anticorps de la population est d'au moins 50%, d'au moins 60%, d'au moins 70%, d'au moins 80%, d'au moins 90%, jusqu'à 100% de galactosylation.

Selon un mode particulier de la population d'anticorps hautement galactosylés, le taux de galactosylation de l'ensemble des anticorps de la population est d'au moins 60%.

Le taux de galactosylation peut être déterminé avec la formule suivante :

$$\frac{\sum_{i=1}^{n} (\text{nombre de Gal}) \quad * \quad (\text{\% surface relative})}{\sum_{i=1}^{n} (\text{nombre de A}) \quad * \quad (\text{\% surface relative})} * 100$$

dans laquelle :

- « n » représente le nombre de pics N-glycanes analysés sur un chromatogramme, par exemple d'un spectre de chromatographie en phase liquide à haute performance en phase normale (NP HPLC),
- « nombre de Gal » représente le nombre de galactoses sur l'antenne du glycanne correspondant au pic,
- « nombre de A » représente le nombre d'antennes N-acétyl-glucosamine de la forme glycannique correspondant au pic, et
- « % surface relative » correspond au pourcentage de l'aire sous le pic correspondant.

Le taux de galactosylation des anticorps de la population d'anticorps peut être déterminé, par exemple, en libérant les N-glycanes des anticorps, en résolvant les N-glycanes sur un chromatogramme, en identifiant le motif d'oligosaccharide du N-glycane qui correspond à un pic spécifique, en déterminant l'intensité du pic et en appliquant les données à la formule mentionnée ci-dessus.

Des anticorps qui sont galactosylés incluent des anticorps qui ont des N-glycanes mono-galactosylés et des N-glycanes bi-galactosylés.

Selon un aspect particulier de la population d'anticorps hautement galactosylés, la population comprend des anticorps qui comprennent des N-glycanes mono-galactosylés, qui peuvent ou non être sialylés. Selon un aspect particulier de la population d'anticorps hautement galactosylés, au moins 1%, au moins 5%, au moins 10%, au moins 15%, au moins 20%, au moins 25%, au moins 30%, au moins 40%, au moins 50%, au moins 60%, au moins 70%, au moins 80%, au moins 90%, jusqu'à 100% des N-glycanes des anticorps comprennent des N-glycanes mono-galactosylés. Selon encore un mode particulier de la demande ou de l'invention, dans la population d'anticorps hautement galactosylés, au moins 25% des anticorps comprennent des N-glycanes mono-galactosylés.

Selon un aspect particulier de la population d'anticorps hautement galactosylés, la population comprend des anticorps qui comprennent des N-glycanes bi-galactosylés, qui peuvent ou non être sialylés. Selon un aspect particulier de la population d'anticorps hautement galactosylés, au moins 1%, au moins 5%, au moins 10%, au moins 15%, au moins 20%, au moins 25%, au moins 30%, au moins 40%, au moins 50%, au moins 60%, au moins 70%, au moins 80%, au moins 90%, jusqu'à 100% des N-glycanes des anticorps comprennent des N-glycanes bi-galactosylés. Selon encore un mode particulier de la demande ou de l'invention, dans la population d'anticorps hautement galactosylés, au moins 35% des anticorps comprennent des N-glycanes bi-galactosylés.

Selon encore un autre aspect de la population d'anticorps hautement galactosylés, la population comprend des anticorps qui comprennent des N-glycanes mono-galactosylés, qui peuvent ou non être sialylés, et des anticorps qui comprennent des N-glycanes bi-galactosylés, qui peuvent ou non être sialylés.

Selon un aspect particulier de la population d'anticorps hautement galactosylés, au moins 1%, au moins 5%,

au moins 10%, au moins 15%, au moins 20%, au moins 25%, au moins 30%, au moins 40%, au moins 50%, au moins 60%, au moins 70%, au moins 80%, au moins 90%, jusqu'à 99% des N-glycanes des anticorps comprennent des N-glycanes mono-galactosylés, et au moins 1%, au moins 5%, au moins 10%, au moins 15%, au moins 20%, au moins 25%, au moins 30%, au moins 40%, au moins 50%, au moins 60%, au moins 70%, au moins 80%, au moins 90%, jusqu'à 99% des N-glycanes des anticorps comprennent des N-glycanes bi-galactosylés.

Selon un autre aspect particulier de la population d'anticorps hautement galactosylés, au moins 25% des anticorps comprennent des N-glycanes mono-galactosylés, et au moins 35% des anticorps comprennent des N-glycanes bi-galactosylés.

Selon encore un autre aspect de la population d'anticorps hautement galactosylés, la population comprend des anticorps hautement fucosylés. Une population d'anticorps hautement fucosylés est une population d'anticorps dans laquelle le taux de fucosylation des N-glycanes des anticorps de la population est d'au moins 40%, au moins 50%, au moins 60%, au moins 70%, au moins 80%, au moins 90%, jusqu'à 100% de fucosylation. Selon un aspect particulier de la population d'anticorps hautement galactosylés, le taux de fucosylation des N-glycanes des anticorps est d'au moins 50%.

Le taux de fucosylation peut être déterminé à l'aide de la formule suivante :

$$\sum_{i=1}^{n} (\text{nombre de Fucose}) \quad * \quad (\%\ \text{surface relative})$$

dans laquelle :

- « n » représente le nombre de pics de N-glycanes analysés sur un chromatogramme, par exemple d'un spectre de chromatographie en phase liquide à haute performance en phase normale (NP HPLC),
- « nombre de Fucose » représente le nombre de fucoses sur la structure glycannique correspondant au pic,
- « % surface relative » correspond au pourcentage de l'aire sous le pic correspondant contenant le fucose.

Des anticorps qui sont fucosylés incluent des anticorps qui ont au moins un monosaccharide fucose sur un de leur N-glycanes. Les anticorps qui sont fucosylés incluent des anticorps qui ont au moins un monosaccharide fucose sur chacun de leur N-glycanes.

Selon un aspect particulier, la population d'anticorps fait référence à une population dans laquelle le taux de galactosylation des N-glycanes des anticorps dans la population est d'au moins 60%, et le taux de fucosylation des anticorps dans la population est d'au moins 50%. Selon un aspect particulier, la population d'anticorps fait référence à une population dans laquelle le taux de galactosylation des N-glycanes des anticorps dans la population est d'au moins 50%, et le taux de fucosylation des N-glycanes des anticorps dans la population est d'au moins 50%, d'au moins 60%, d'au moins 70%, d'au moins 80%, d'au moins 90%, jusqu'à 100%.

Selon encore un autre aspect particulier, la population d'anticorps décrits ici, fait référence à une population dans laquelle le taux de galactosylation des N-glycanes des anticorps dans la population est d'au moins 60%, et le taux de fucosylation des N-glycanes des anticorps dans la population est d'au moins 50%, d'au moins 60%, d'au moins 70%, d'au moins 80%, d'au moins 90%, jusqu'à 100%.

Selon encore un autre aspect particulier, la population d'anticorps décrits ici, fait référence à une population dans laquelle le taux de galactosylation des N-glycanes des anticorps dans la population est d'au moins 70%, et le taux de fucosylation des N-glycanes des anticorps dans la population est d'au moins 50%, d'au moins 60%, d'au moins 70%, d'au moins 80%, d'au moins 90%, jusqu'à 100%.

Selon encore un autre aspect particulier, la population d'anticorps décrits ici, fait référence à une population dans laquelle le taux de galactosylation des N-glycanes des anticorps dans la population est d'au moins 80%, et le taux de fucosylation des N-glycanes des anticorps dans la population est d'au moins 60%, d'au moins 70%, d'au moins 80%, d'au moins 90%, jusqu'à 100%.

Selon encore un autre aspect particulier, la population d'anticorps décrits ici, fait référence à une population dans laquelle le taux de galactosylation des N-glycanes des anticorps dans la population est d'au moins 90%, et le taux de fucosylation des N-glycanes des anticorps dans la population est d'au moins 60%, d'au moins 70%, d'au moins 80%, d'au moins 90%, jusqu'à 100%.

Selon encore un autre aspect particulier, la population d'anticorps décrits ici, fait référence à une population dans laquelle le taux de galactosylation des N-glycanes des anticorps dans la population est jusqu'à 100%, et le taux de fucosylation des N-glycanes des anticorps dans la population est d'au moins 60%, d'au moins 70%, d'au moins 80%, d'au moins 90%, jusqu'à 100%.

Selon encore un autre aspect de la demande ou de l'invention, l'invention concerne une composition comprenant une population d'anticorps dirigés soit contre une cytokine pro-inflammatoire circulante, soit contre une toxine bactérienne

circulante avec un rapport spécifique entre les pourcentages des N-glycanes des anticorps dans la population qui sont galactosylés sur le site de galactosylation du fragment Fc gamma et les pourcentages des N-glycanes des anticorps dans la population qui sont fucosylés sur le site de glycosylation du fragment Fc gamma.

Selon un aspect particulier, la demande ou l'invention concerne une composition comprenant une population d'anticorps dirigés soit contre une cytokine pro-inflammatoire circulante, soit contre une toxine bactérienne circulante dans laquelle le rapport entre le taux de galactosylation des N-glycanes des anticorps dans la population et le taux de fucosylation des N-glycanes des anticorps dans la population est entre 0,5 et 2,5 ; entre 0,6 et 2,0 ; entre 0,7 et 1,8 ou entre 1,0 et 1,4.

Selon encore un autre aspect particulier, la demande ou l'invention concerne une composition comprenant une population d'anticorps dans laquelle le rapport entre le taux de galactosylation des N-glycanes des anticorps dans la population et le taux de fucosylation des N-glycanes des anticorps dans la population est entre 1,0 et 1,4, par exemple 1,2.

Selon encore un autre aspect, les anticorps dirigés soit contre une cytokine pro-inflammatoire circulante, soit contre une toxine bactérienne circulante ont été modifiés pour contenir un oligomannose ou un oligomannose supplémentaire. Dans un autre mode de réalisation, les anticorps ont été modifiés de sorte qu'au moins 30 % des anticorps contiennent au moins un oligomannose. Dans un autre mode de réalisation, les anticorps ont été modifiés de sorte qu'au moins 40 %, 50%, 60%, 70 %, 80%, 90% des anticorps contiennent au moins un oligomannose. Dans un autre mode de réalisation, les anticorps ont été modifiés de telle sorte que moins de 50%, 40 %, 30%, 20 %, 10%, des anticorps contiennent du fucose sur au moins une chaine de l'anticorps. Dans encore un autre mode de réalisation, les anticorps ont été modifiés de sorte qu'au moins 40% , 50%, 60 %, 70 %, 80% , 90% des anticorps contient au moins un oligomannose et moins de 50 %, 40% , 30 %, 20 %, 10%, des anticorps contiennent du fucose sur au moins une chaîne de l'anticorps.

Dans un autre mode de réalisation, les N-glycanes des anticorps dirigés soit contre une cytokine pro-inflammatoire circulante, soit contre une toxine bactérienne circulante ont été modifiés pour présenter un profil de glycosylation hautement mannosylé. Dans encore un autre mode de réalisation, les anticorps ont été modifiés de sorte qu'au moins une chaîne de l'anticorps contient un oligomannose et est non fucosylée. Dans encore un autre mode de réalisation, les anticorps ont été modifiés de sorte que le N-glycane majeur des anticorps est non fucosylé. Dans un mode de réalisation le principal glucide est un oligomannose non fucosylés. Dans un autre mode de réalisation, le principal N-glycane est un Man5 non fucosylé. Dans encore un autre mode de réalisation, les anticorps ont été modifiés de telle sorte que moins de 40% des hydrates de carbone des anticorps contiennent du fucose. Dans encore un autre mode de réalisation, les anticorps ont été modifiés de telle sorte que moins de 30%, 20 %, 10% des hydrates de carbone des anticorps contiennent du fucose. Dans un mode de réalisation le fucose est le 1,6 -fucose. Dans un autre mode de réalisation, les anticorps ont été modifiés de sorte qu'au moins 60% des N-glycanes des anticorps sont un oligomannose non fucosylé et moins de 40% des hydrates de carbone sont des anticorps contenant du fucose.

Dans encore un autre mode de réalisation, les anticorps sont modifiés de telle sorte que les N-glycanes des anticorps présentent un profil de glycosylation hautement mannosylé. Dans un mode de réalisation les anticorps sont modifiés de telle sorte qu'au moins une chaîne d'anticorps contient un oligomannose et est non fucosylée. Dans un autre mode de réalisation, les anticorps sont modifiés de telle sorte qu'une chaîne d'anticorps contient un oligomannose et est non fucosylée. Dans un autre mode de réalisation, les anticorps sont modifiés de telle sorte que le N-glycane majeur des anticorps est non fucosylé. Dans un mode de réalisation le principal N-glycane est un oligomannose non fucosylé. Dans un autre mode de réalisation, le principal N-glycane est un Man5 non fucosylé. Dans encore un autre mode de réalisation, les anticorps sont modifiés de telle sorte que moins de 40% des N-glycanes des anticorps contiennent du fucose. Dans un mode de réalisation les anticorps sont modifiés de telle sorte que moins de 30%, 20 %, 10% ou moins des hydrates de carbone contiennent des anticorps fucose. Dans encore un autre mode de réalisation, les anticorps sont modifiés de sorte qu'au moins 30 % des anticorps possèdent au moins un oligomannose. Dans un mode de réalisation les anticorps sont modifiés de sorte qu'au moins 40 %, 50%, 60%, 70 %, 80%, 90% des anticorps présentent au moins un oligomannose. Dans encore un autre mode de réalisation, les anticorps sont modifiés de sorte qu'au moins 40 %, 50% , 60%, 70 %, 80% , 90% des anticorps contiennent au moins un oligomannose et moins de 50 %, 40% , 30 %, 20 %, 10% , des anticorps contiennent un fucose sur au moins une chaîne. Dans un autre mode de réalisation, les anticorps sont modifiés de sorte qu'au moins 60% des N-glycanes des anticorps sont un oligomannose non-fucosylé et moins de 40% des N-glycanes des anticorps contiennent du fucose.

L'expression « profil de glycosylation hautement mannosylé » est destinée à désigner un anticorps qui contient au moins un oligomannose ou une composition d'anticorps dans laquelle au moins 30 % des anticorps contiennent au moins un oligomannose. Dans certains modes de réalisation au moins 30 %, 40 %, 50%, 60 %, 70%, 80 %, 90% des N-glycanes des anticorps sont un oligomannose. Dans certains modes de réalisation au moins 30 %, 40 %, 50%, 60 %, 70%, 80 %, 90% des N-glycanes des anticorps sont un oligomannose non - fucosylé. Dans d'autres formes de réalisation moins de 50 %, 40% , 30 %, 20% , 10 %, 5% des N-glycanes des anticorps contiennent du fucose. Dans d'autres modes de réalisation, les anticorps sont faibles en fucose et riches en oligomannose. Par conséquent, dans

d’autres modes de réalisation, au moins 30% , 40 %, 50% , 60%, 70 %, 80% ou 90% des N-glycanes des anticorps sont un oligomannose et moins de 50% , 40 %, 30% , 20 %, 10% ou 5% des N-glycanes des anticorps contiennent du fucose. Par conséquent, dans encore un autre mode de réalisation , au moins 30%, 40 %, 50% , 60%, 70 %, 80% ou 90% ou plus des N-glycanes des anticorps sont un oligomannose non- fucosylé et moins de 50 %, 40% , 30 %, 20 %, 10% ou 5% des N-glycanes sont des anticorps contenant du fucose. Un mode de réalisation de la demande ou de l’invention se rapporte à des anticorps avec une liaison au récepteur Fcgamma RIII trouvé sur les monocytes, les macrophages et les cellules tueuses naturelles accrue, qui ne disposent pas d’un sucre 1,6- fucose sur la chaîne lourde.

La demande ou l’invention concerne également une composition d’anticorps dirigés soit contre une cytokine pro-inflammatoire circulante, soit contre une toxine bactérienne circulante dans laquelle les anticorps contiennent un oligommanose.

La demande ou l’invention concerne également une composition dans laquelle au moins 30% des anticorps contiennent au moins un oligomannose.

La demande ou l’invention concerne également une composition dans laquelle les N-glycanes des anticorps présentent un profil de glycosylation hautement mannosylé.

La demande ou l’invention concerne également une composition dans laquelle au moins une chaine des anticorps contient un oligomannose et n’est pas fucosylée.

La demande ou l’invention concerne également une composition dans laquelle le N-glycane majeur des anticorps n’est pas fucosylé.

La demande ou l’invention concerne également une composition dans laquelle le N-glycane majeur des anticorps est un oligomannose non-fucosylé.

La demande ou l’invention concerne également une composition dans laquelle le N-glycane majeur des anticorps est un Man5 non-fucosylé.

La demande ou l’invention concerne également une composition dans laquelle moins de 40% des N-glycanes des anticorps contiennent du fucose.

La demande ou l’invention concerne également une composition dans laquelle les anticorps ne contiennent pas de fucose.

La demande ou l’invention concerne également une composition dans laquelle au moins 60% des N-glycanes des anticorps contiennent un oligomannose fucosylé et dans laquelle moins de 40% des N-glycanes des anticorps contiennent du fucose.

Les anticorps peuvent être produits par les techniques décrites dans la demande internationale WO/2007/048077 ou encore dans la demande provisoire américaine 61,065 du 13 février 2013.

Des profils d’anticorps sont également décrits dans la demande internationale WO/2007/048077 ou encore dans la demande provisoire américaine 61,065 du 13 février 2013.

De façon avantageuse, les anticorps ont une activité de cytoxicité dépendante du complément (CDC) élevée et/ou une activité de cytotoxicité à médiation cellulaire dépendante des anticorps (ADCC) élevée.

La demande ou l’invention sera mieux illustrée par les exemples et figures suivantes. Les exemples ci-après visent à éclaircir l’objet de la demande ou de l’invention et illustrer des modes de réalisation avantageux.

**Légende des figures :**

**Figure 1 :** Chromatogramme représentant les N-glycanes d’une population d’anticorps adalimumab hautement galactosylés issus d’une chèvre #1.

**Figure 2:** Chromatogramme représentant les N-glycanes d’une population d’anticorps adalimumab hautement galactosylés issus d’une chèvre #1 à 7 jours de lactation.

**Figure 3:** Chromatogramme représentant les N-glycanes d’une population d’anticorps adalimumab hautement galactosylés issus d’une chèvre #1 à 17 jours de lactation.

**Figure 4:** Chromatogramme représentant les N-glycanes d’une population d’anticorps adalimumab hautement galactosylés issus d’une chèvre #1 à 32 jours de lactation.

**Figure 5 :** Résumé des pourcentages des oligosaccharides des N-glycanes des populations d’anticorps adalimumab hautement galactosylés issus d’une chèvre #1 à divers jours de lactation.

**Figure 6:** Chromatogramme représentant les N-glycanes d’une population d’anticorps adalimumab hautement galactosylés issus d’une chèvre #2 à 3 jours de lactation.

**Figure 7:** Chromatogramme représentant les N-glycanes d’une population d’anticorps adalimumab hautement galactosylés issus d’une chèvre #2 à 11 jours de lactation.

**Figure 8:** Chromatogramme représentant les N-glycanes d’une population d’anticorps adalimumab hautement galactosylés issus d’une chèvre #2 à 21 jours de lactation.

**Figure 9 :** Résumé des pourcentages des oligosaccharides des N-glycanes des populations d'anticorps adalimumab hautement galactosylés issus d'une chèvre #2 à divers jours de lactation.
**Figure 10** : Anticorps adalimumab produit par transgénèse se liant au TNF-α soluble.
**Figure 11** : Anticorps adalimumab produit par transgénèse se liant au CD16 exprimé sur les cellules NK après compétition avec un anticorps 3G8 anti-CD16.
**Figure 12** : Anticorps adalimumab produit par transgénèse se liant au TNF- α soluble et au exprimé par les cellules de Jurkat alors qu'une version aglycosylé de l'anticorps adalimumab produit par transgénèse ne se lie pas.
**Figure 13** : correspond à la représentation de la capacité à induire la phagocytose du TNF soluble par les macrophages CD16+.

**EXEMPLES :**

**Matériels et méthodes des Exemples 1 et 2**

*Génération de chèvres transgéniques produisant l'adalimumab*

Des chèvres transgéniques ont été produites par l'introduction dans leur génome de la séquence d'acide nucléique codant pour l'anticorps de l'adalimumab. Les chèvres produisant l'adalimumab ont été générées en utilisant des techniques traditionnelles de micro-injection (c.f. US 7,928,064). L'ADNc codant pour la chaîne lourde et légère (SEQ ID NO: 3 et SEQ ID NO: 4) est synthétisée sur la base de la séquence d'acides aminés publiée (brevet US 6,090,382). Ces séquences d'ADN ont été liguées avec le vecteur d'expression de la caséine bêta pour donner les constructions BC2601 HC et LC BC2602. Dans ces plasmides, la séquence d'acide nucléique codant pour l'adalimumab est sous le contrôle d'un promoteur facilitant l'expression de l'adalimumab dans la glande mammaire des chèvres. Les séquences procaryotes ont été enlevées et l'ADN a été micro-injecté dans des embryons pré-implantatoires de la chèvre. Ces embryons ont ensuite été transférés à des femelles pseudo gravides. La descendance en résultant a été criblée pour la présence des transgènes. Ceux qui portaient les deux chaînes ont été identifiés comme fondateurs transgéniques.
A l'âge approprié, les animaux fondateurs ont été élevés. Après la grossesse et la parturition, ils ont été nourris au lait. Une cinétique de lactation en jours a été réalisée à partir de la parturition (par exemple, jour 3, jour 7, jour 11). L'anticorps adalimumab a été purifié à partir du lait à chaque point de temps et caractérisé tel que décrit ici.

*Mesure de la liaison de l'adalimumab produit par transgénèse par la méthode ELISA:*

Le TNF-α a été coaté pendant une nuit à 4°C dans une plaque 96 puits sous 100 μl de PBS à une concentration de 5 μg/ml. Après blocage des sites aspécifiques (incubation avec 200 μl de PBS/BSA à 1%, 1 h à température ambiante), l'adalimumab produit par transgénèse ou l'adalimumab déglycosylée a été ajouté à diverses concentrations (de 0 à 10 μg/ml) pendant 20 minutes dans du PBS/BSA à 1%. Après lavage, la liaison de l'adalimumab produite par transgénèse au TNF-α a été évaluée par l'addition d' un anticorps de chèvre anti-IgG humaine (H+L) couplé à la peroxydase, suivi par le substrat ($H_2O_2$ et tétraméthylbenzidine). Après 20 min d'incubation, la réaction a été arrêtée avec 50 μl de $H_2SO_4$ dilué, et la DO a été lue à 450 nm. Les résultats pour l'adalimumab produit par transgénèse sont présentés à la figure 10.

*Liaison au CD16, compétition avec l'anticorps 3G8*

Pour évaluer la liaison de l'adalimumab produit par transgénèse au CD 16, une étude de déplacement de la liaison avec l'anticorps anti-CD16 3G8, (Santa Cruz Biotech) a été réalisée. L'essai de déplacement a permis de déterminer l'efficacité de la liaison de l'adalimumab produit par transgénèse au récepteur CD16 exprimé à la surface de la membrane des cellules NK.
Les cellules tueuses naturelles (cellules NK) ont été purifiées par appauvrissement négatif (Miltenyi) à partir du sang périphérique de donneurs sains. Les cellules NK ont ensuite été incubées à des concentrations variables de l'adalimumab produit par transgénèse (de 0 à 83 μg/ml) et à une concentration fixe de l'anticorps anti-CD16 3G8 conjugué à un fluorochrome (3G8-PE). Après lavage, la liaison de 3G8-PE au récepteur sur les cellules NK a été évaluée par cytométrie en flux. Les valeurs moyennes de fluorescence (VMF) observées ont été exprimées en pourcentage de liaison; une valeur de 100% correspond à la valeur observée sans l'adalimumab produit par transgénèse et qui correspond donc à la liaison maximale de 3G8. Une valeur de 0% correspond à la VMF en l'absence d'anticorps 3G8. L'$IC_{50}$, c'est-à-dire la concentration d'anticorps nécessaire pour induire une inhibition de 50 % de Imax de la liaison de 3G8, a été calculé en utilisant le logiciel PRISM. Les résultats sont présentés à la figure 11.

*Liaison du TNF-α soluble avec l'adalimumab produit par transgénèse au CD16 exprimé sur les cellules Jurkat par l'intermédiaire du fragment Fc de l'adalimumab produit par transgénèse*

Des cellules Jurkat-CD16 ont été incubées avec 10 μg/ml de l'adalimumab produit par transgénèse ou la version déglycosylée de celui-ci pendant 20 min à 4°C. Après lavage, 100 μl de TNF-α ont été ajoutés sur le culot cellulaire à la concentration finale de 1 μg/ml, pendant 20 min à 4°C. Après un lavage supplémentaire, les cellules ont été incubées avec 5 μg/ml d'un anticorps de chèvre biotinylé anti-TNF-α humain, pendant 20 min à 4°C. Après un autre cycle de lavage, la liaison du TNF-α a été visualisée par l'addition de streptavidine couplée au fluorochrome-PE pendant 20 min à 4°C. Les échantillons ont été analysés par cytométrie en flux. Les résultats sont présentés à la figure 12.

**Résultats :**

**Exemple 1** : adalimumab produit par transgénèse

Le profil de glycosylation des anticorps adalimumab produits dans le lait de chèvres transgéniques a été déterminé par la libération des N-glycanes de l'anticorps et de l'analyse sur colonne des oligosaccharides ainsi libérés Les Figures **1-4** et **6-8** montrent les oligosaccharides (N-glycanes) libérés de l'anticorps adalimumab produit par transgénèse issu de la chèvre #1 (figures 1-4) et de la chèvre #2 (figures 6-8). Les groupes de monosaccharides sont représentés comme suit:

Carré noir : la N-acétylglucosamine (GlcNAc)

Triangle : fucose

Cercle gris : mannose

Cercle blanc : galactose

Diamant Gris: acide N- glycolylneuraminique (NGNA) : un acide sialique

Diamant blanc: acide N-acétylneuraminique (NANA) : un acide sialique

La Figure **1** montre un chromatogramme représentant les oligosaccharides (N-glycanes) libérés de l'anticorps adalimumab produit par transgénèse dans le lait de la chèvre #1. Le chromatogramme montre que parmi les quatorze principaux oligosaccharides (N-glycanes) produits, douze possèdent au moins un galactose dans la chaîne N-glycannique, dont quatre oligosaccharides possèdent deux galactoses. Seulement deux des oligosaccharides sont de purs oligomannoses (Voir le pic 1 et le pic 3). La figure 1 montre également que parmi les quatorze principaux oligosaccharides produits, neuf sont fucosylés. Tous les oligosaccharides fucosylés sont également galactosylés.

Les Figures **2-4** montrent les chromatogrammes d'oligosaccharides (N-glycanes) libérés de l'anticorps adalimumab produit par transgénèse dans le lait de chèvre #1 tel que récolté après 7 jours de lactation (figure 2), 17 jours de lactation (figure 3), et 32 jours de lactation (figure 4).

Les pourcentages relatifs de l'ensemble des N-glycanes isolés à partir de l'anticorps adalimumab produit dans le lait de chèvre #1 sont illustrés à la Figure **5.** La Figure **5** montre également un tableau du pourcentage global de mono-galactosylation, du pourcentage de bi-galactosylation, du pourcentage de galactosylation totale (mono-galactosylation + bi-galactosylation), le pourcentage de galactosylation a été calculé conformément à la formule indiquée ci-dessus, le pourcentage de fucosylation a été calculé conformément à la formule indiquée ci-dessus, du rapport galactosylation sur fucosylation et du pourcentage de structures glycanniques possédant au moins un acide sialique (% de sialylation). Les résultats sont également résumés dans le tableau 1 ci-dessous :

Table 1 : oligosaccharides (N-glycanes) isolés à partir des anticorps adalimumab de la chèvre #1

| | jour 7 | jour 17 | jour 32 | moyenne |
|---|---|---|---|---|
| mono-Gal (%): | 30,8 | 42,9 | 44,1 | 39,2 |
| bi-Gal (%): | 53,1 | 46,0 | 47,0 | 48,7 |
| mono-Gal + bi-Gal (%) | 83,9 | 88,9 | 91,1 | 88,0 |
| Gal* (%) | 82,9 | 88,2 | 89,8 | 87,0 |
| Fuc* (%) | 63,5 | 74,9 | 81,9 | 73,4 |

(suite)

| | jour 7 | jour 17 | jour 32 | moyenne |
|---|---|---|---|---|
| Rapport Gal/Fuc | 1,30 | 1,17 | 1,10 | 1,18 |
| Silaylation (%) | 50,4 | 59,3 | 62,7 | 57,5 |
| *calculés selon les formules figurant dans la description | | | | |

Les Figures **6-8** montrent les chromatogrammes d'oligosaccharides (N-glycanes) libérés de l'anticorps adalimumab produit par transgénèse dans le lait de la chèvre # 2 tel que récolté après 3 jours de lactation (figure 6), 11 jours de lactation (figure 7), et 21 jours de lactation (figure 8).

Les pourcentages relatifs de l'ensemble des oligosaccharides (N-glycanes) isolés à partir de l'anticorps adalimumab produit dans le lait de chèvre # 2 sont illustrés à la Figure **9.**

La Figure **9** montre également un tableau du pourcentage global de mono-galactosylation, du pourcentage de bi-galactosylation, du pourcentage de galactosylation totale (mono-galactosylation + bi-galactosylation), le pourcentage de galactosylation a été calculé conformément à la formule indiquée ci-dessus, le pourcentage de fucosylation a été calculé conformément à la formule indiquée ci-dessus, du rapport galactosylation sur fucosylation et du pourcentage de structures glycanniques possédant au moins un acide sialique (% de sialylation). Les résultats sont également résumés dans le tableau 2 ci-dessous:

Table 2 : oligosaccharides (N-glycanes) isolés à partir des anticorps adalimumab de la chèvre #2

| | jour 3 | jour 11 | jour 21 | moyenne |
|---|---|---|---|---|
| mono-Gal (%): | 27,3 | 25,7 | 27,5 | 26,8 |
| bi-Gal (%): | 39,0 | 43,0 | 31,4 | 37,8 |
| mono-Gal + bi-Gal (%) | 66,3 | 68,7 | 58,9 | 64,6 |
| Gal* (%) | 64,6 | 67,9 | 57,8 | 63,4 |
| Fuc* (%) | 51,6 | 54,0 | 46,0 | 50,5 |
| Gal/Fuc | 1,25 | 1,25 | 1,25 | 1,25 |
| Sialylation (%) | 40,8 | 42,6 | 39,1 | 40,8 |
| *calculés selon les formules figurant dans la description | | | | |

**Exemple 2** : Etudes de liaison de l'adalimumab produit par transgénèse

La Figure **10** montre que l'adalimumab produit par transgénèse peut lier le TNF-alpha soluble coaté dans des plaques 96 puits. L'adalimumab produit par transgénèse et qui est non-glycosylé est également capable de lier le TNF-alpha soluble (données non présentées).

La Figure **11** montre que l'adalimumab produit par transgénèse lie le exprimé par les cellules tueuses naturelles (NK). La liaison a été démontrée dans une expérience de compétition avec l'anticorps anti-CD16 3G8. La liaison de l'adalimumab produit par transgénèse à est plus forte que la liaison d'un anticorps faiblement galactosylé au CD16 (données non présentées).

La Figure **12** montre que les anticorps adalimumab produits par transgénèse lient à la fois le TNF-alpha soluble et les cellules Jurkat exprimant alors que la version non glycosylé de l'anticorps adalimumab produit par transgénèse en est incapable.

**Exemple 3** : Test de phagocytose

**Matériel de l'Exemple 3**

Les réactifs suivants ont été utilisés :

- anti-TNF transgénique Humira,
  - non déglycosylé (c'est-à-dire glycosylé)
  - déglycosylé
- anti-TNF Humira, (Adalimumab, Abbott)

- Immunoglobulines polyvalentes IVIg (Tégéline, LFB)

Des monocytes isolés à partir de sang périphérique ont été décongelés et différenciés en macrophages pendant 3 jours dans du RPMI 1640 + 10% SVF + M-CSF à 50 ng / ml. TNF-$\alpha$ a été marqué avec le kit Lightning-Link Rapid Conjugaison System Innova Biosciences (fluorescence verte) selon les instructions du fabricant puis incubés pendant 20 minutes à 4°C avec 10 $\mu$g/ml d'anticorps anti-TNF alpha.
La phagoctose a été réalisée pendant 3 heures à 4°C et 37°C en incubant le TNF- $\alpha$ marqué avec les macrophages (1.105 cellules/puits) en présence ou en l'absence d'1mg/ml d'immunoglobulines IVIg
L'indice de phagocytose analysé par cytométrie de flux est estimé selon la formule suivante : MFI 37°C - MFI 4°C (Unité Arbitraire).
Les résultats sont présentés dans la Figure **13.**
Les macrophages seuls (contrôle négatif) montrent une absence de fluorescence à 4°C et 37°C.
En présence de l'anticorps adalimumab transgénique non déglycosylé (TG-Humira), de TNF-$\alpha$ et de macrophages, la valeur de phagocytose est de 15 (MFI).
Dans les mêmes conditions, l'ajout d'IVIg (1mg/ml) induit une inhibition de la fixation de l'anticorps TG-Humira aux macrophages (FcR). Ceci est observé à 4°C et à 37°C. La phagocytose est estimée à 13.3 (MFI).
En présence de l'anticorps Humira d'Abbott (Humira commercial) de TNF-$\alpha$ et de macrophages, la valeur de phagocytose est de 12.5 (MFI).
Dans les mêmes conditions l'ajout d'IVIg (1mg/ml) induit une inhibition de la fixation de l'anticorps Humira d'Abbott aux macrophages (FcR). Ceci est observé à 4°C et à 37°C. La phagocytose est estimée à 6.72 (MFI).
Ces résultats montrent donc que l'anticorps TG-Humira induit une phagocytose du TNF-$\alpha$ en présence de macrophages CD16+ supérieure à celle induite par l'anticorps adalimumab commercial (Humira, Abbott).

## Revendications

1. Composition comprenant des anticorps monoclonaux dirigés contre une cytokine pro-inflammatoire circulante, lesdits anticorps ayant une affinité au moins égale à $2x10^7$ $M^{-1}$ pour le récepteur Fc$\gamma$RIIIa (CD16) et dans laquelle le taux de fucose de l'ensemble des anticorps de ladite composition est inférieur à 60%, pour son utilisation dans le cadre de la prévention ou du traitement de l'inflammation, notamment dans laquelle lesdits anticorps ont une affinité au moins égale à $2x10^8$ $M^{-1}$ ou $2x10^9$ $M^{-1}$, telle que déterminée par l'analyse Scatchard ou la technologie BIAcore (Label-free surface plasmon resonance based technology).

2. Composition pour son utilisation selon la revendication 1, le taux de fucose de l'ensemble des anticorps de ladite composition étant inférieur à 50%.

3. Composition pour son utilisation selon la revendication 1 ou 2, **caractérisée en ce que** chacun des anticorps possède sur le site de glycosylation en position 297 de ses chaines lourdes, l'une des formes glycanniques biantennées choisie parmi les structures suivantes :

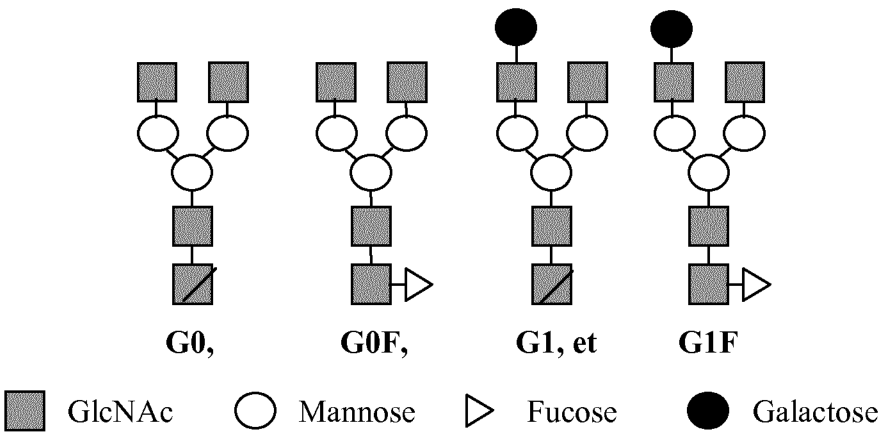


le GlcNAc représenté par 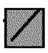 dans les structures G0 et G1 ci-dessus étant susceptible d'être fucosylé.

4. Composition pour son utilisation selon la revendication 3, **caractérisée en ce que** les formes G0F + G1F des anticorps de ladite composition représentent moins de 50% des structures glycanniques portées par le site de glycosylation en position 297 de la chaîne lourde (Asn 297), notamment dans laquelle les formes G0 + G1 + G0F + G1F des anticorps de ladite composition représentent plus de 60% desdites structures glycaniques et de préférence plus de 80% des structures glycanniques portées par le site de glycosylation en position 297 de la chaîne lourde (Asn 297).

5. Composition pour son utilisation selon l'une quelconque des revendications 1 à 4, **caractérisée en ce que** chaque anticorps monoclonal compris dans ladite composition possède une affinité pour les récepteurs FcyRIII au moins 1,5 fois supérieure à celle d'un anticorps naturel dirigé contre ladite cytokine pro-inflammatoire circulante, de préférence ledit anticorps ne possédant pas de propriétés de neutralisation de ladite cytokine pro-inflammatoire circulante, et ladite cytokine pro-inflammatoire étant notamment choisie parmi les cytokines pro-inflammatoires suivantes :

   a. TNF-$\alpha$,
   b. IL-1$\beta$,
   c. IL-6,
   d. IL-8,
   e. IL-10,
   f. IL-12,
   g. IL-17
   h. IL-18
   i. GM-CSF.

6. Composition pour son utilisation selon l'une quelconque des revendications 1 à 5, **caractérisée en ce que** ledit anticorps est utilisé à des doses variant de 0,05 mg/m$^2$ à 2000 mg/m$^2$, notamment sous forme injectable ou sous forme de spray, et de préférence en association avec au moins un agent anti inflammatoire.

7. Composition pour son utilisation selon la revendication 1, dans laquelle le taux de galactosylation de l'ensemble des anticorps de la population est d'au moins 60%, notamment d'au moins 70%, et de préférence d'au moins 80%.

8. Composition pour son utilisation selon la revendication 7, dans laquelle le taux de fucosylation de l'ensemble des anticorps de la population est d'au moins 50%.

9. Composition pour son utilisation selon la revendication 7, dans laquelle la population comprend des anticorps qui comprennent des N-glycanes mono-galactosylés et/ou des N-glycanes bi-galactosylés, notamment dans laquelle au moins 35% des anticorps dans la population comprennent des N-glycanes bi-galactosylés et au moins 25% des anticorps dans la population comprennent des N-glycanes mono-galactosylés.

10. Composition pour son utilisation selon l'une quelconque des revendications 7 à 9, dans laquelle le rapport du taux de galactosylation des anticorps de la population et du taux de fucosylation des anticorps de la population est compris de 1,0 à 1,4.

11. Composition pour son utilisation selon l'une quelconque des revendications 1 à 10, dans laquelle l'anticorps est produit dans les cellules épithéliales mammaires d'un mammifère non-humain, notamment un mammifère non-humain transgénique, en particulier une chèvre, un mouton, un bison, un chameau, une vache, un cochon, un lapin, un buffle, un cheval, un rat, une souris ou un lama.

12. Composition pour son utilisation selon l'une quelconque des revendications 1 à 11, comprenant un véhicule pharmaceutiquement acceptable, ladite composition pouvant également comprendre du lait.

13. Composition pour son utilisation selon la revendication 1 dans laquelle les anticorps contiennent un oligommanose, de préférence au moins 30% des anticorps contiennent au moins un oligomannose, et notamment dans laquelle les anticorps ne contiennent pas de fucose.

14. Composition pour son utilisation selon la revendication 13 dans laquelle les N-glycanes des anticorps contiennent au moins un oligomannose.

15. Composition pour son utilisation selon la revendication 14 dans laquelle au moins une chaine des anticorps contient

un oligomannose et n'est pas fucosylée.

16. Composition pour son utilisation selon la revendication 13 dans laquelle le N-glycane majeur des anticorps n'est pas fucosylé, de préférence le N-glycane majeur des anticorps étant un oligomannose non-fucosylé, notamment un Man5 non-fucosylé.

17. Composition pour son utilisation selon la revendication 13 dans laquelle moins de 40% des N-glycanes des anticorps contiennent du fucose, de préférence dans laquelle au moins 60% des N-glycanes des anticorps contiennent un oligomannose non fucosylé et dans laquelle moins de 40% des N-glycanes des anticorps contiennent du fucose.

**Patentansprüche**

1. Zusammensetzung, umfassend monoklonale Antikörper, die gegen ein zirkulierendes entzündungsförderndes Cytokin gerichtet sind, wobei die Antikörper eine Affinität von mindestens gleich $2x10^7$ $M^{-1}$ für den Rezeptor FcγRIIIa (CD16) aufweisen und wobei der Fucoseanteil der Gesamtheit der Antikörper der Zusammensetzung kleiner als 60 % ist, zur Verwendung im Rahmen der Prävention oder der Behandlung der Entzündung, wobei die Antikörper insbesondere eine Affinität von mindestens gleich $2x10^8$ $M^{-1}$ oder $2x10^9$ $M^{-1}$ aufweisen, wie mittels Scatchard-Analyse oder die BIAcore-Technologie (markerfreie Oberflächen-Plasmon-Resonanzbasierte Technologie) bestimmt.

2. Zusammensetzung zur Verwendung nach Anspruch 1, wobei der Fucoseanteil der Gesamtheit der Antikörper der Zusammensetzung kleiner als 50 % ist.

3. Zusammensetzung zur Verwendung nach Anspruch 1 oder 2, **dadurch gekennzeichnet, dass** jeder der Antikörper an der Glycosylierungsstelle an Position 297 seiner schweren Ketten eine der zweiantennigen Glycanformen aufweist, ausgewählt aus den folgenden Strukturen:

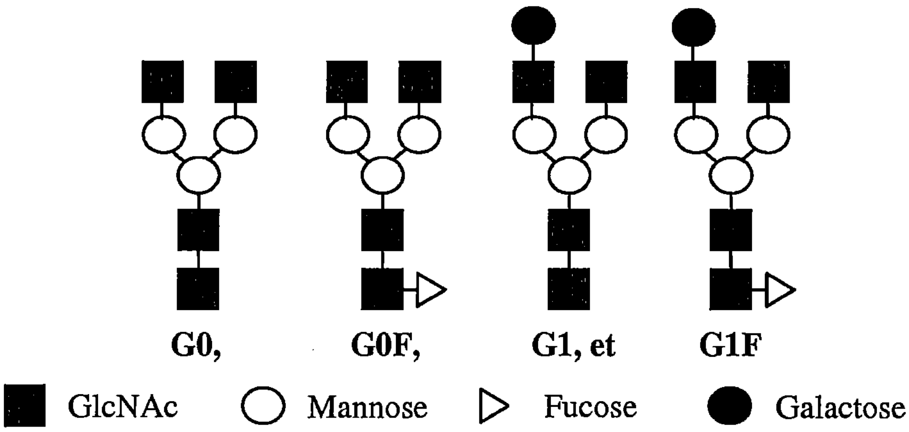


wobei das GlcNAc, das in den Strukturen G0 und G1 oben durch ■ dargestellt ist, fucosyliert werden kann.

4. Zusammensetzung zur Verwendung nach Anspruch 3, **dadurch gekennzeichnet, dass** die Formen G0F + G1F der Antikörper der Zusammensetzung weniger als 50 % der Glycanstrukturen ausmachen, die von der Glycosylierungsstelle an Position 297 der schweren Kette (Asn 297) getragen werden, wobei die Formen G0 + G1 + G0F + G1F der Antikörper der Zusammensetzung mehr als 60 % der Glycanstrukturen und vorzugsweise mehr als 80 % der Glycanstrukturen ausmachen, die von der Glycosylierungsstelle an Position 297 der schweren Kette (Asn 297) getragen werden.

5. Zusammensetzung zur Verwendung nach einem der Ansprüche 1 bis 4, **dadurch gekennzeichnet, dass** jeder monoklonale Antikörper, der in der Zusammensetzung enthalten ist, eine Affinität für die FcγRIII-Rezeptoren aufweist, die mindestens 1,5-mal größer ist als die eines natürlichen Antikörpers, der gegen das zirkulierende entzündungsfördernde Cytokin gerichtet ist, wobei die Antikörper der Zusammensetzung vorzugsweise keine Neutralisationseigenschaften des zirkulierenden entzündungsfördernden Cytokins aufweisen, und wobei das entzündungsfördernde Cytokin insbesondere aus den folgenden entzündungsfördernden Cytokinen ausgewählt ist:

a. TNP-$\alpha$,
b. IL-1$\beta$,
c. IL-6,
d. IL-8,
e. IL-10,
f. IL-12,
g. IL-17
h. IL-18
i. GM-CSF.

6. Zusammensetzung zur Verwendung nach einem der Ansprüche 1 bis 5, **dadurch gekennzeichnet, dass** der Antikörper in Dosen verwendet wird, die im Bereich von 0,05 mg/$m^2$ bis 2.000 mg/$m^2$ liegen, insbesondere in injizierbarer Form oder in Sprayform, und insbesondere in Verbindung mit mindestens einem entzündungshemmenden Mittel.

7. Zusammensetzung zur Verwendung nach Anspruch 1, wobei der Galactosylierungsgrad der Gesamtheit der Antikörper der Population mindestens 60 %, insbesondere mindestens 70 % und vorzugsweise mindestens 80 % beträgt.

8. Zusammensetzung zur Verwendung nach Anspruch 7, wobei der Fucosylierungsgrad der Gesamtheit der Antikörper der Population mindestens 50 % beträgt.

9. Zusammensetzung zur Verwendung nach Anspruch 7, wobei die Population Antikörper umfasst, die monogalactosylierte N-Glycane und/oder bigalactosylierte N-Glycane umfassen, wobei insbesondere mindestens 35 % der Antikörper in der Population bigalactosylierte N-Glycane umfassen und mindestens 25 % der Antikörper in der Population monogalactosylierte N-Glycane umfassen.

10. Zusammensetzung zur Verwendung nach einem der Ansprüche 7 bis 9, wobei das Verhältnis des Galactosylierungsgrades der Antikörper der Population zum Fucosylierungsgrad der Antikörper der Population zwischen 1,0 und 1,4 liegt.

11. Zusammensetzung zur Verwendung nach einem der Ansprüche 1 bis 11, wobei der Antikörper in den Brustepithelzellen eines nicht-humanen Säugetiers, insbesondere eines transgenen nicht-humanen Säugetiers, insbesondere einer Ziege, einem Schaf, einem Bison, einem Kamel, einer Kuh, einem Schwein, einem Kaninchen, einem Büffel, einem Pferd, einer Ratte, einer Maus oder einem Lama, produziert wird.

12. Zusammensetzung zur Verwendung nach einem der Ansprüche 1 bis 11, umfassend ein pharmazeutisch verträgliches Vehikel, wobei die Zusammensetzung auch Milch umfassen kann.

13. Zusammensetzung zur Verwendung nach Anspruch 1, wobei die Antikörper eine Oligomannose enthalten, wobei mindestens 30 % der Antikörper vorzugsweise mindestens eine Oligomannose enthalten, und wobei die Antikörper insbesondere keine Fucose enthalten.

14. Zusammensetzung zur Verwendung nach Anspruch 13, wobei die N-Glycane der Antikörper mindestens eine Oligomannose enthalten.

15. Zusammensetzung zur Verwendung nach Anspruch 14, wobei mindestens eine Kette der Antikörper eine Oligomannose enthält und nicht fucosyliert ist.

16. Zusammensetzung zur Verwendung nach Anspruch 13, wobei das Haupt-N-Glycan der Antikörper nicht fucosyliert ist, wobei das Haupt-N-Glycan der Antikörper vorzugsweise eine nicht fucosylierte Oligomannose, insbesondere eine nicht fucosylierte Man5 ist.

17. Zusammensetzung zur Verwendung nach Anspruch 13, wobei weniger als 40 % der N-Glycane der Antikörper Fucose enthalten, wobei vorzugsweise weniger als 60 % der N-Glycane der Antikörper eine nicht fucosylierte Oligomannose enthalten und wobei weniger als 40 % der N-Glycane der Antikörper Fucose enthalten.

## Claims

1. Composition comprising monoclonal antibodies directed against a circulating proinflammatory cytokine, said antibodies having an affinity at least equal to $2x10^7$ $M^{-1}$ for the FcγRIIIa receptor (CD16) and wherein the fucose level of all of the antibodies of said composition is less than 60%, for the use thereof in the context of the prevention or treatment of the early phases of inflammation,
in particular wherein said antibodies having an affinity at least equal to $2x10^8$ $M^{-1}$ or $2x10^9$ $M^{-1}$, as determined by Scatchard analysis or BIAcore technology (Label-free surface plasmon resonance based technology).

2. Composition for its use according to claim 1, the fucose level of all of the antibodies of said composition being less than 50%.

3. Composition for its use according to claim 1 or 2, **characterized in that** each of the antibodies has, on the glycosylation site in position 297 of its heavy chains, one of the biantennary glycan forms selected from the following structures:

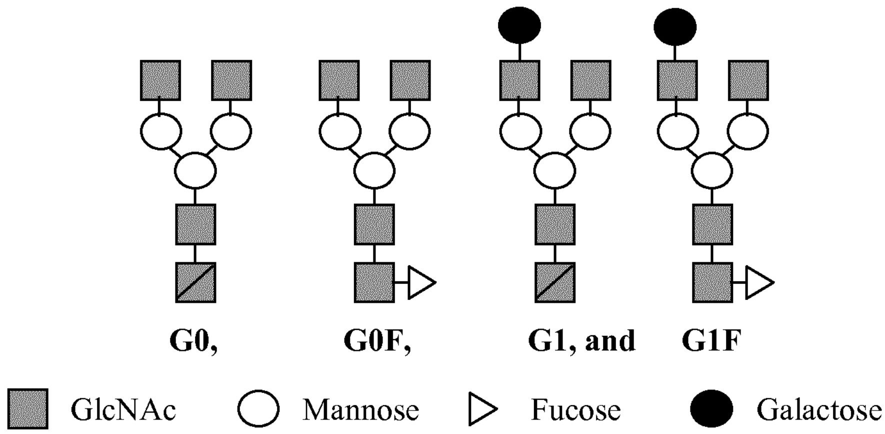


the GlcNAc represented by 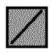 in the above G0 and G1 structures being capable of being fucosylated.

4. Composition for its use according to claim 3, **characterized in that** the G0F + G1F forms of the antibodies of said composition represent less than 50% of the glycan structures borne by the glycosylation site in position 297 of the heavy chain (Asn 297), in particular wherein the G0 + G1+ G0F + G1F forms of the antibodies of said composition represent more than 60% of said glycan structures and preferably more than 80% of the glycan structures borne by the glycosylation site in position 297 of the heavy chain (Asn 297).

5. Composition for its use according to any one of claims 1 to 4, **characterized in that** each monoclonal antibody comprised in said composition has an affinity for the FcγRIII receptors at least 1.5 times greater than that of a natural antibody directed against said circulating proinflammatory cytokine, preferably said antibody of the composition has no properties of neutralization of said circulating proinflammatory cytokine, and said proinflammatory cytokine is in particular selected from the following proinflammatory cytokines:

    a. TNF-α,
    b. IL-1β,
    c. IL-6,
    d. IL-8,
    e. IL-10,
    f. IL-12,
    g. IL-17
    h. IL-18
    i. GM-CSF.

6. Composition for its use according to any one of claims 1 to 5, **characterized in that** said antibody is used in doses varying from 0.05 $mg/m^2$ to 2000 $mg/m^2$, in particular in injectable form or in spray form, and preferably combined with at least one anti-inflammatory agent.

7. Composition for its use according to claim 1, wherein the galactosylation level of all of the antibodies of the population is at least 60%, in particular at least 70%, and preferably at least 80%.

8. Composition for its use according to claim 7, wherein the fucosylation level of all of the antibodies of the population is at least 50%.

9. Composition for its use according to claim 7, wherein the population comprises antibodies which comprise mono-galactosylated N-glycans and/or bi-galactosylated N-glycans, in particular wherein at least 35% of the antibodies in the population comprise bi-galactosylated N-glycans and at least 25% of the antibodies in the population comprise mono-galactosylated N-glycans.

10. Composition for its use according to any one of claims 7 to 9, wherein the ratio of the galactosylation level of the antibodies of the population to the fucosylation level of the antibodies of the population is from 1.0 to 1.4.

11. Composition for its use according to any one of claims 1 to 10, wherein the antibody is produced in the mammary epithelial cells of a non-human mammal, in particular in a transgenic non-human mammal, in particular in a goat, a sheep, a bison, a camel, a cow, a pig, a rabbit, a buffalo, a horse, a rat, a mouse or a llama.

12. Composition for its use according to any one of claims 1 to 11, comprising a pharmaceutically acceptable vehicle, said composition could also comprising milk.

13. Composition for its use according to claim 1, wherein the antibodies contain an oligommanose, preferably at least 30% of the antibodies contain at least one oligomannose, and wherein in particular said antibodies contain no fucose.

14. Composition for its use according to claim 13, wherein the N-glycans of the antibodies contain at least one oligomannose.

15. Composition for its use according to claim 14, wherein at least one chain of the antibodies contains an oligomannose and is not fucosylated.

16. Composition for its use according to claim 13, wherein the major N-glycan of the antibodies is not fucosylated, preferably the major N-glycan of the antibodies is a non-fucosylated oligomannose, in particular a non-fucosylated Man5.

17. Composition for its use according to claim 13, wherein less than 40% of the N-glycans of the antibodies contain fucose, preferably wherein at least 60% of the N-glycans of the antibodies contain a non-fucosylated oligomannose and wherein less than 40% of the N-glycans of the antibodies contain fucose.

FIGURE 1

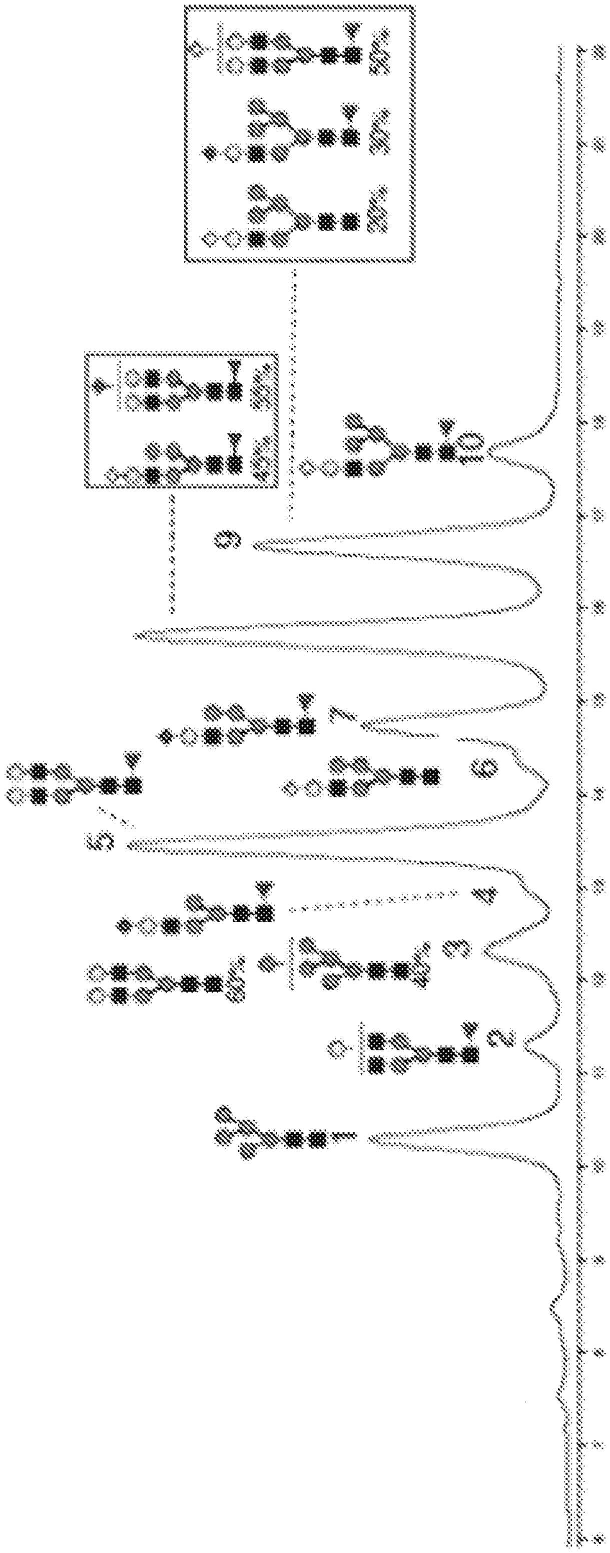

**FIGURE 2**

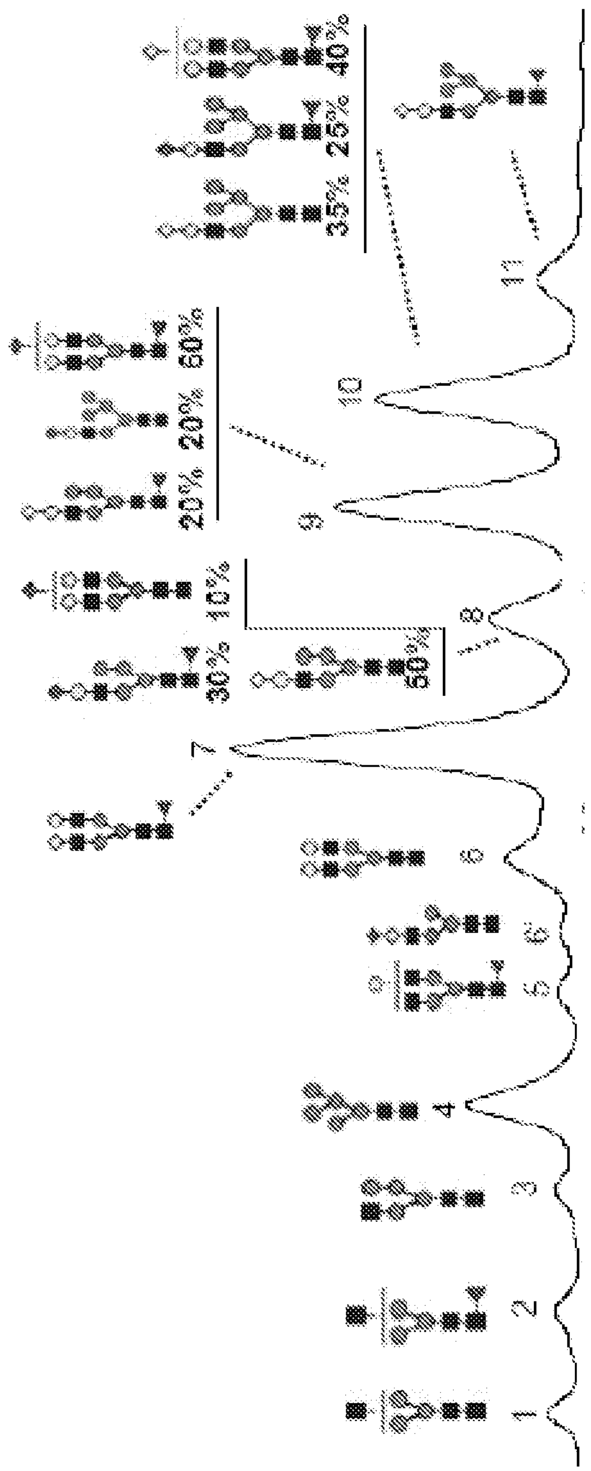

# FIGURE 3

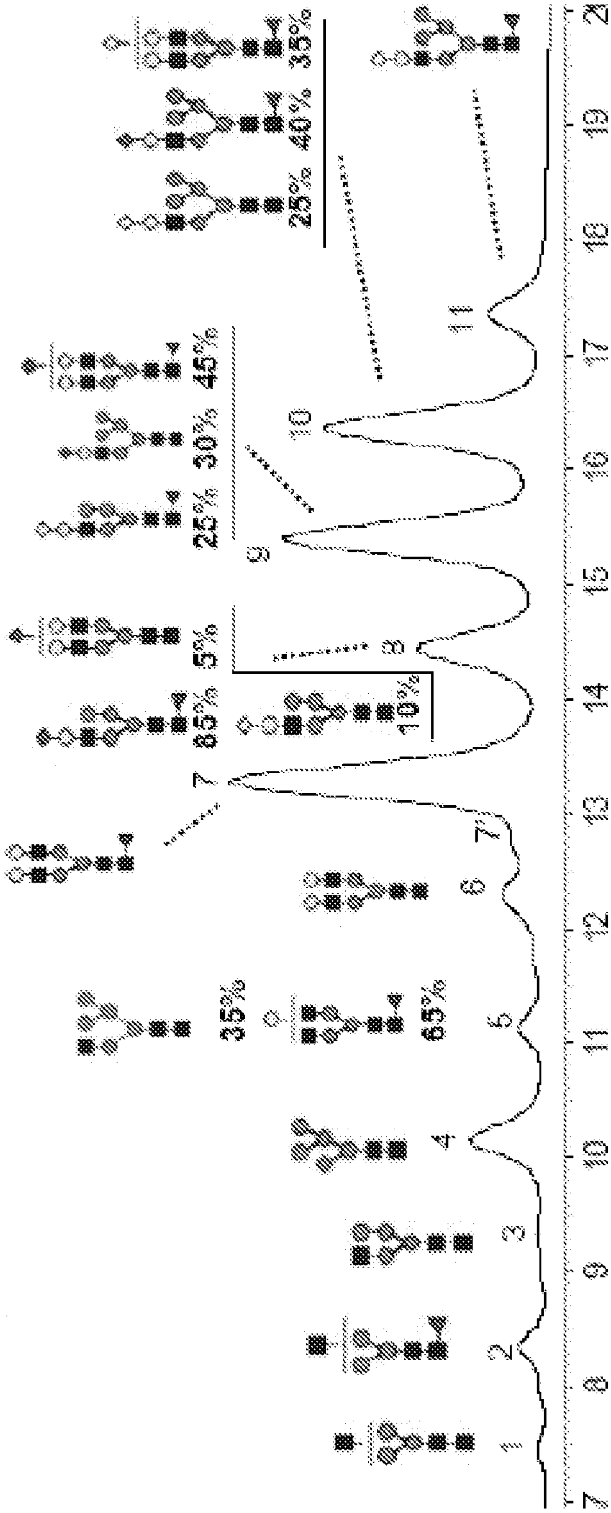
1
2
3
4
5
35%
65%
6
7'
7
85%
10%
5%
8
9
25%
30%
45%
10
11
25%
40%
35%
7
8
9
10
11
12
13
14
15
16
17
18
19
2

**FIGURE 4**

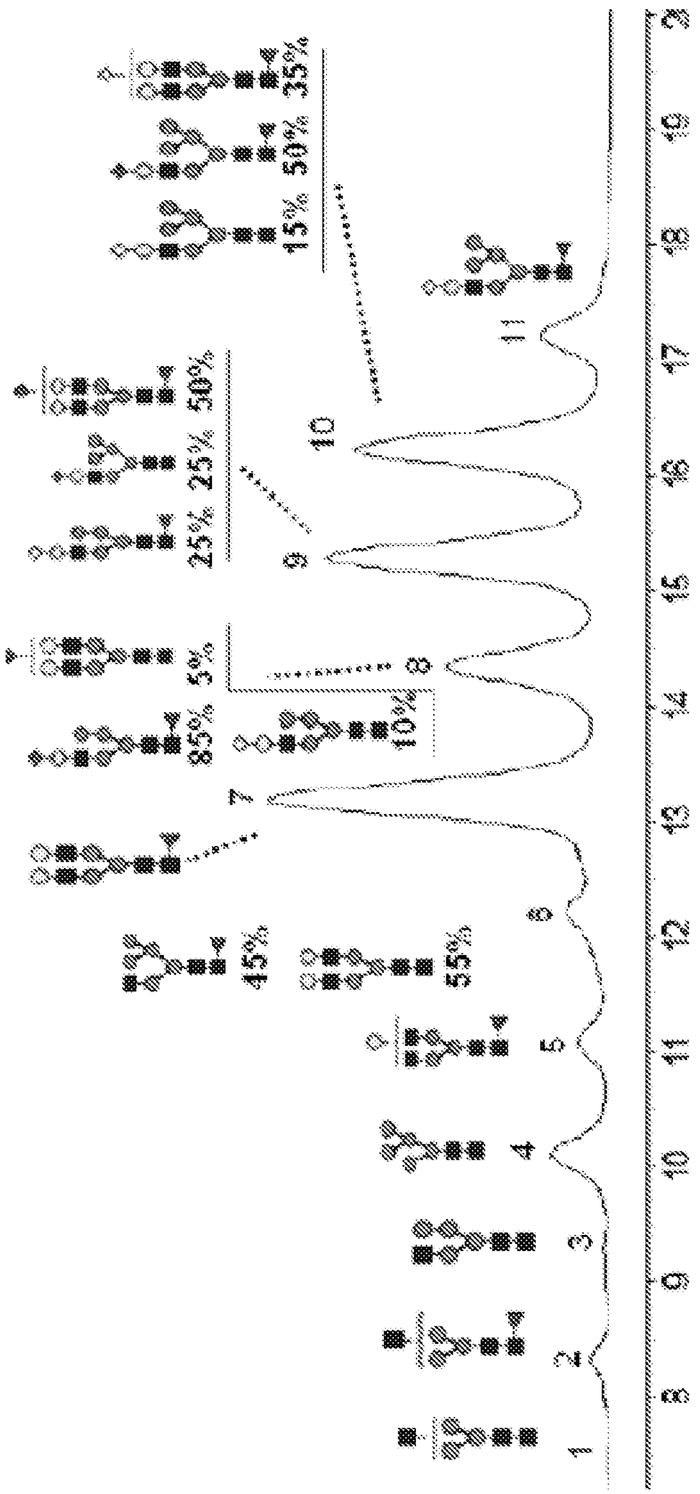
1
2
3
4
5
6
7
8
9
10
11
45%
55%
85%
10%
5%
25%
25%
50%
15%
50%
35%
8
9
10
11
12
13
14
15
16
17
18
19
20

# FIGURE 5

## adalimumab produit dans la chèvre #1

| | Jour 7 | | | Jour 17 | | | Jour 32 | | | | | |
|---|---|---|---|---|---|---|---|---|---|---|---|---|
| RT | Peak | Mass | % | Peak | Mass | % | Peak | Mass | % | Theor | Notes | Structure |
| 7.4 | 1 | 1233.3 | 2.6 | 1 | 1233.3 | 0.8 | 1 | 1233.5 | 0.5 | 1233.5 | | 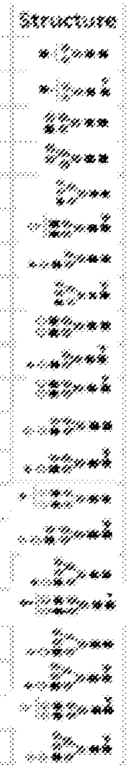 |
| 8.3 | 2 | 1379.4 | 2.2 | 2 | 1379.4 | 2.3 | 2 | 1379.4 | 1.6 | 1379.5 | | |
| 9.3 | 3 | 1395.3 | 2.2 | 3 | 1395.5 | 0.8 | 3 | 1395.5 | 0.5 | 1395.5 | | |
| 10.1 | 4 | 1354.3 | 9.2 | 4 | 1354.3 | 6.4 | 4 | 1354.3 | 4.6 | 1354.5 | | |
| 11 | 5 | | | 5 | 1557.6 | 0.8 | 5 | 1557.5 | trace | 1557.6 | | |
| | | 1744.3 | 2.0 | | 1744.5 | 1.4 | | 1744.5 | 2.6 | 1744.6 | | |
| 11.6 | 6' | 1686.3 | 1.8 | | | | | | | 1686.6 | | |
| 12.3 | 6 | | | 6 | | | 6 | 1703.4 | 1.5 | 1703.6 | | |
| | | 1760.5 | 6.0 | | 1760.5 | 3.7 | | 1760.5 | 1.9 | 1760.6 | | |
| 12.7 | | | | 7 | 1832.5 | 3.0 | | | | 1832.7 | | |
| 13.2 | 7 | 1906.6 | 27.9 | 7 | 1906.2 | 25.9 | 7 | 1906.5 | 26.5 | 1906.7 | | |
| 14.3 | 8 | 1864.5 | 3.7 | 8 | 1864.5 | 1.1 | 8 | 1864.5 | 1.3 | 1864.6 | | |
| | | 1994.5 | 2.2 | | 1994.5 | 9.0 | | 1994.5 | 10.9 | 1994.7 | | |
| | | 2051.5 | 1.5 | | 2051.6 | 0.5 | | 2051.6 | 0.6 | 2051.7 | | |
| 15.4 | 9 | 2010.5 | 3.9 | 9 | 2010.5 | 5.3 | 9 | 2010.5 | 5.5 | 2010.7 | | |
| | | | 3.9 | | | 6.5 | | | 5.5 | | | |
| | | 2197.5 | 11.6 | | 2197.5 | 9.6 | | 2197.5 | 11.0 | 2197.7 | | |
| 16.3 | 10 | 2026.5 | 5.7 | 10 | 2026.5 | 4.5 | 10 | 2026.3 | 3.0 | 2026.7 | | |
| | | 2156.5 | 4.0 | | 2156.6 | 7.2 | | 2156.6 | 10.0 | 2156.8 | | |
| | | 2213.5 | 6.5 | | 2213.5 | 6.3 | | 2213.5 | 7.0 | 2213.8 | | |
| 17.3 | 11 | 2172.5 | 3.6 | 11 | 2172.5 | 4.9 | 11 | 2172.5 | 5.3 | 2172.8 | | |

| | Jour 7 | Jour 17 | Jour 32 | |
|---|---|---|---|---|
| mono-Gal (%): | 30.8 | 42.9 | 44.1 | av: 39.2 |
| bi-Gal (%): | 53.1 | 46.0 | 47.0 | av: 48.7 |
| mono-Gal + bi-Gal (%) | 83.9 | 88.9 | 91.1 | av: 88.0 |
| Gal* (%) | 82.9 | 88.2 | 89.8 | av: 87.0 |
| Fuc* (%) | 63.5 | 74.9 | 81.9 | av: 73.4 |
| Ratio Gal/Fuc | 1.30 | 1.17 | 1.10 | av: 1.18 |
| Sialylation (%) | 50.4 | 59.3 | 62.7 | av: 57.5 |

*calculés selon les formules de la description

FIGURE 6

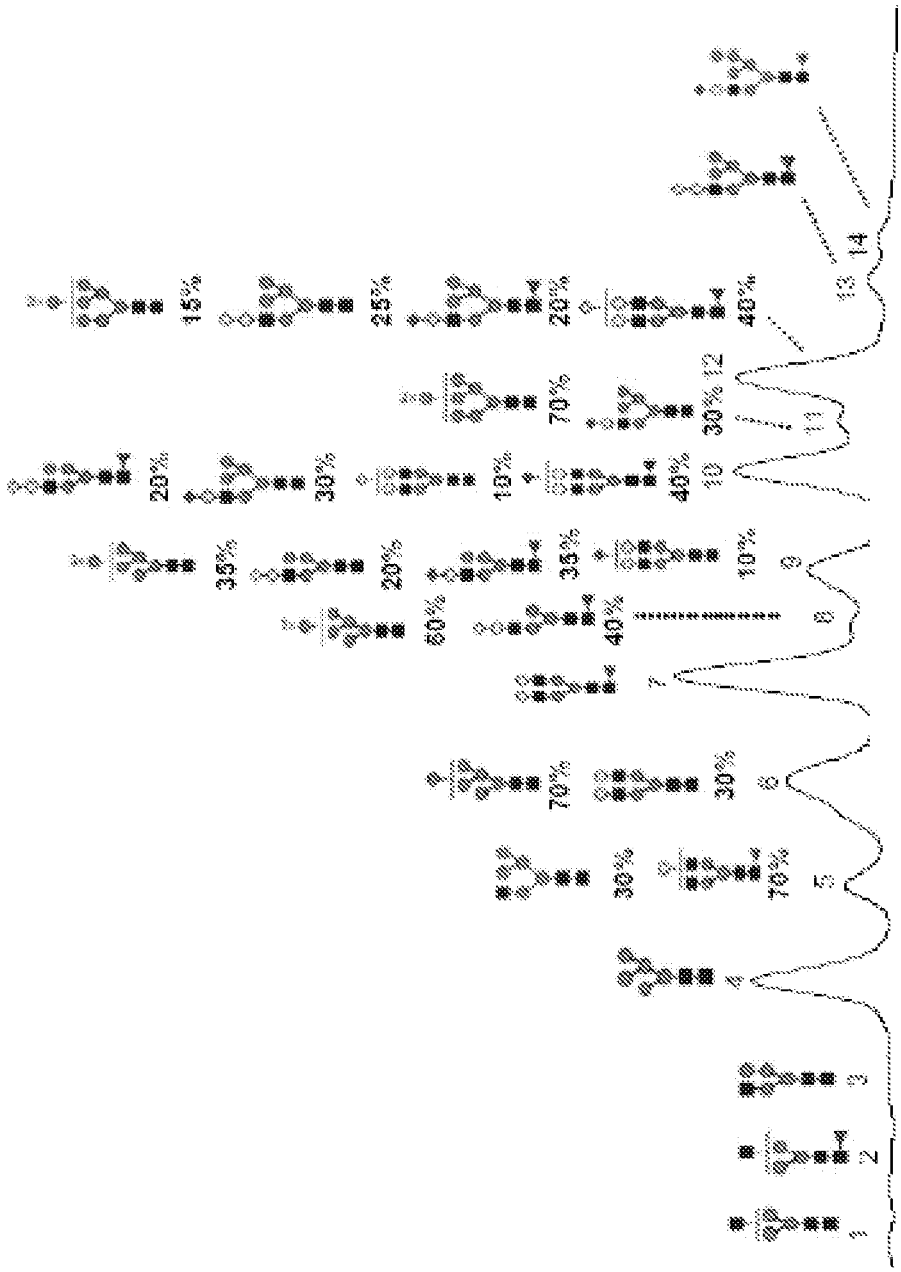

1
2
3
4
30%
70%
5
70%
30%
6
7
60%
40%
8
35%
20%
35%
10%
9
20%
30%
10%
40%
10
70%
30%
11
12
15%
25%
20%
40%
13
14

# FIGURE 7

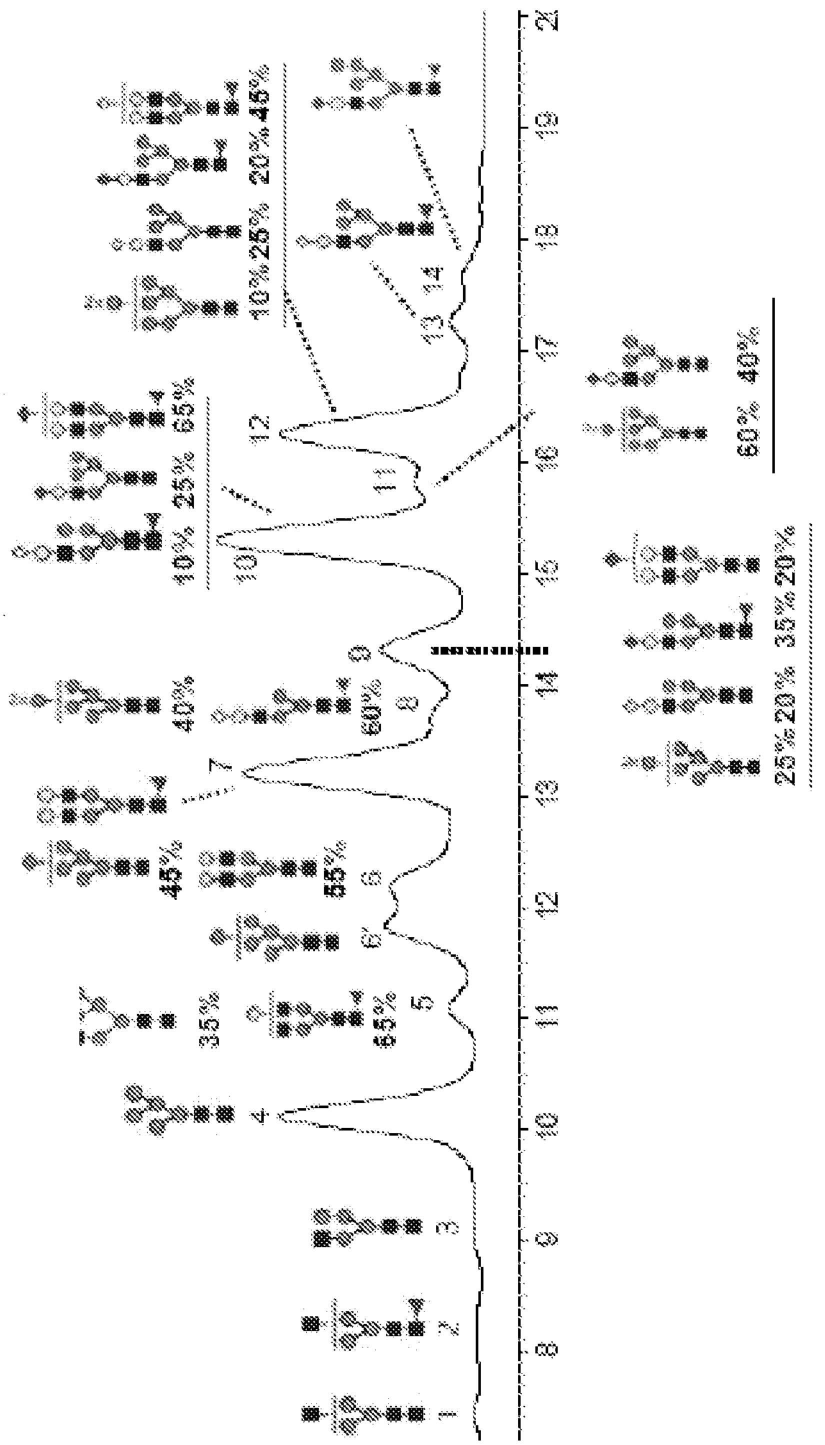

**FIGURE 8**

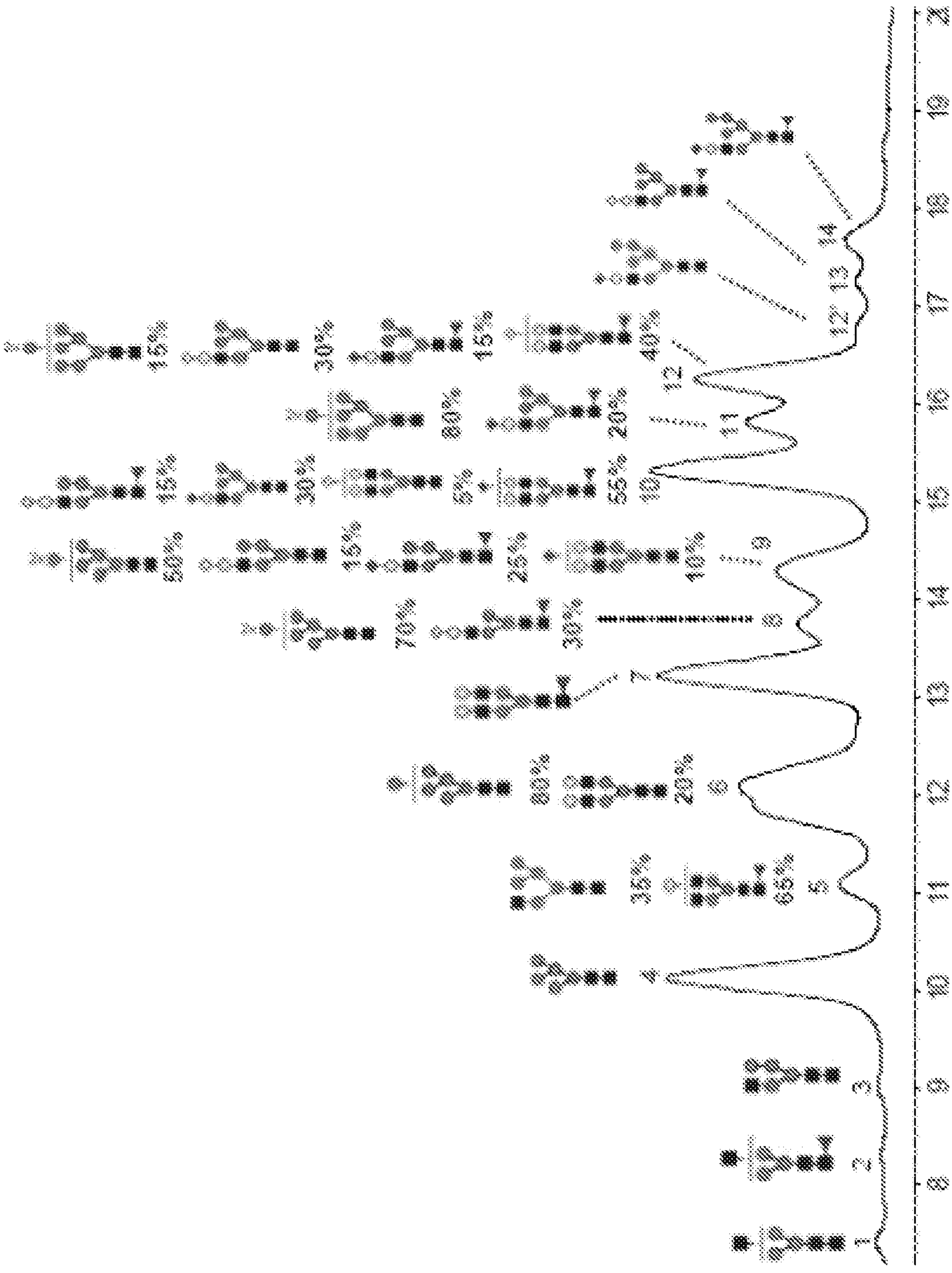

1
2
3
4
35%
65%
5
80%
20%
6
7
70%
30%
8
50%
15%
25%
10%
9
15%
30%
5%
55%
10
80%
20%
11
15%
30%
15%
40%
12
12
13
14
8
9
10
11
12
13
14
15
16
17
18
19
20

FIGURE 9

## Adalimumab produit dans la chèvre #2

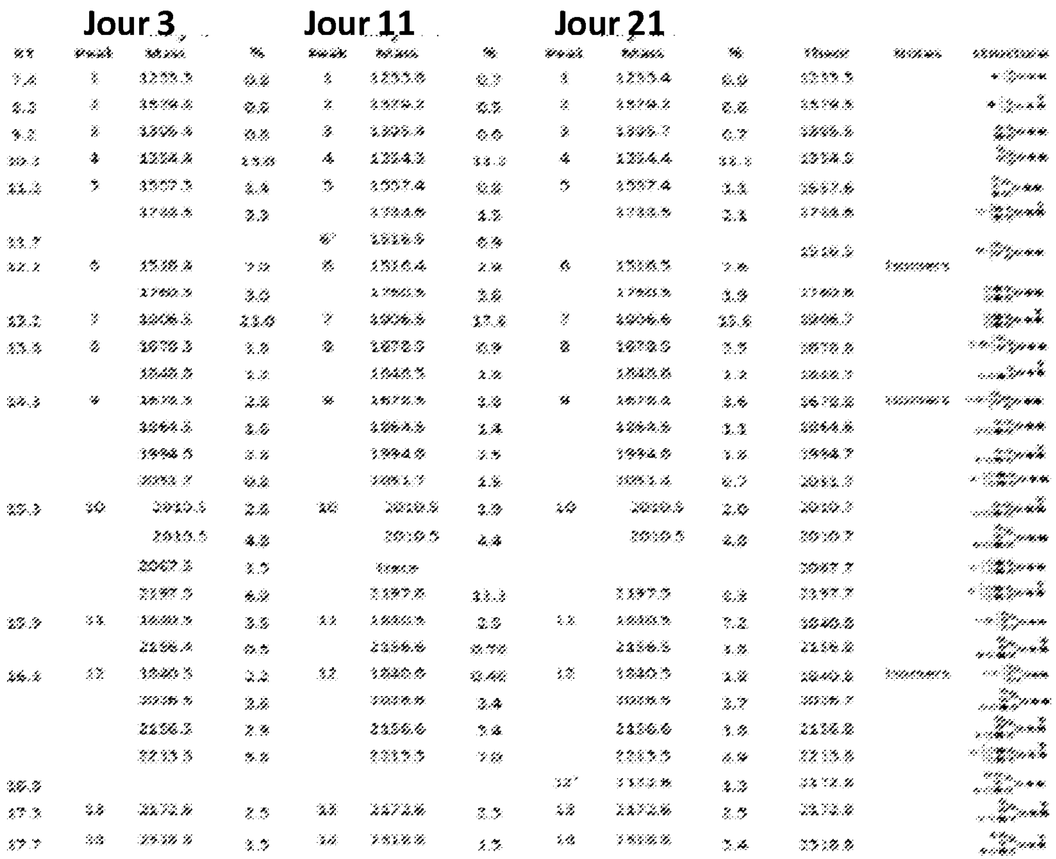


| | Jour 3 | Jour 11 | Jour 21 | |
|---|---|---|---|---|
| mono-Gal (%): | 27.3 | 25.7 | 27.5 | av: 26.8 |
| bi-Gal (%): | 39.0 | 43.0 | 31.4 | av: 37.8 |
| mono-Gal + bi-Gal (%) | 66.3 | 68.7 | 58.9 | av: 64.6 |
| Gal* (%) | 64.6 | 67.9 | 57.8 | av: 63.4 |
| Fuc* (%) | 51.6 | 54.0 | 46.0 | av: 50.5 |
| Gal / Fuc | 1.25 | 1.25 | 1.25 | av: 1.25 |
| Sialylation (%) | 40.8 | 42.6 | 39.1 | av: 40.8 |

*calculés selon les formules de la description

**FIGURE 10**

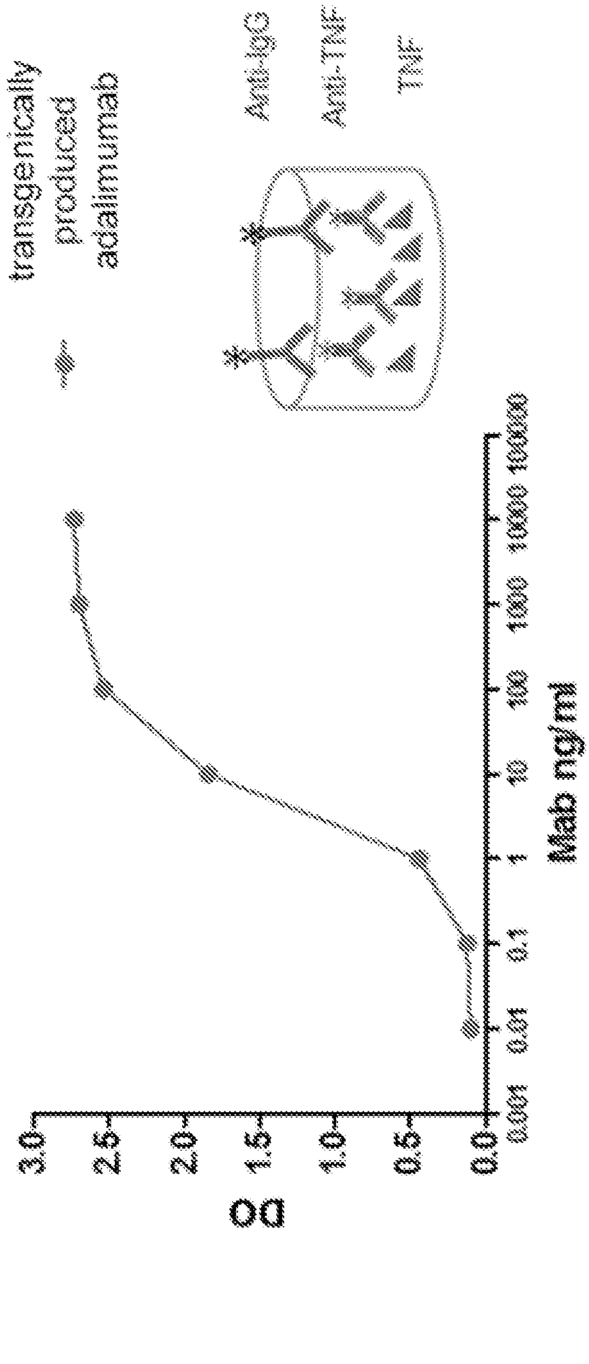
transgenically produced adalimumab
Anti-IgG
Anti-TNF
TNF
DO
3.0
2.5
2.0
1.5
1.0
0.5
0.0
0.001
0.01
0.1
1
10
100
1000
10000
100000
Mab ng/ml

FIGURE 11

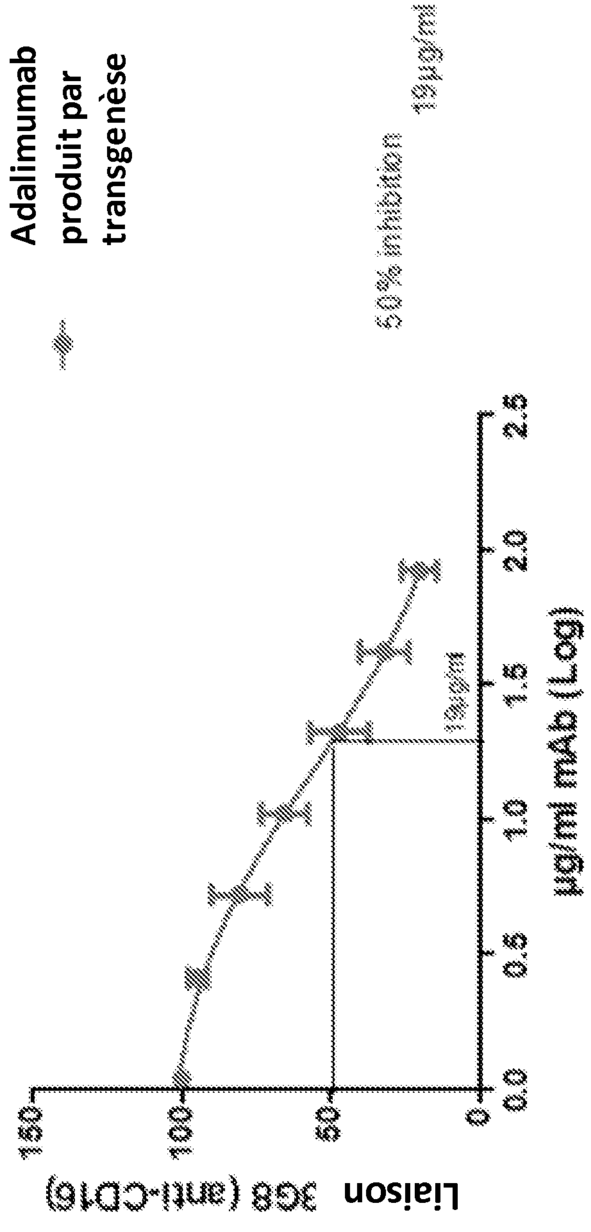
Adalimumab produit par transgenèse
50% inhibition
19µg/ml
Liaison 3G8 (anti-CD16)
150
100
50
0
0.0
0.5
1.0
1.5
2.0
2.5
µg/ml mAb (Log)

**FIGURE 12**

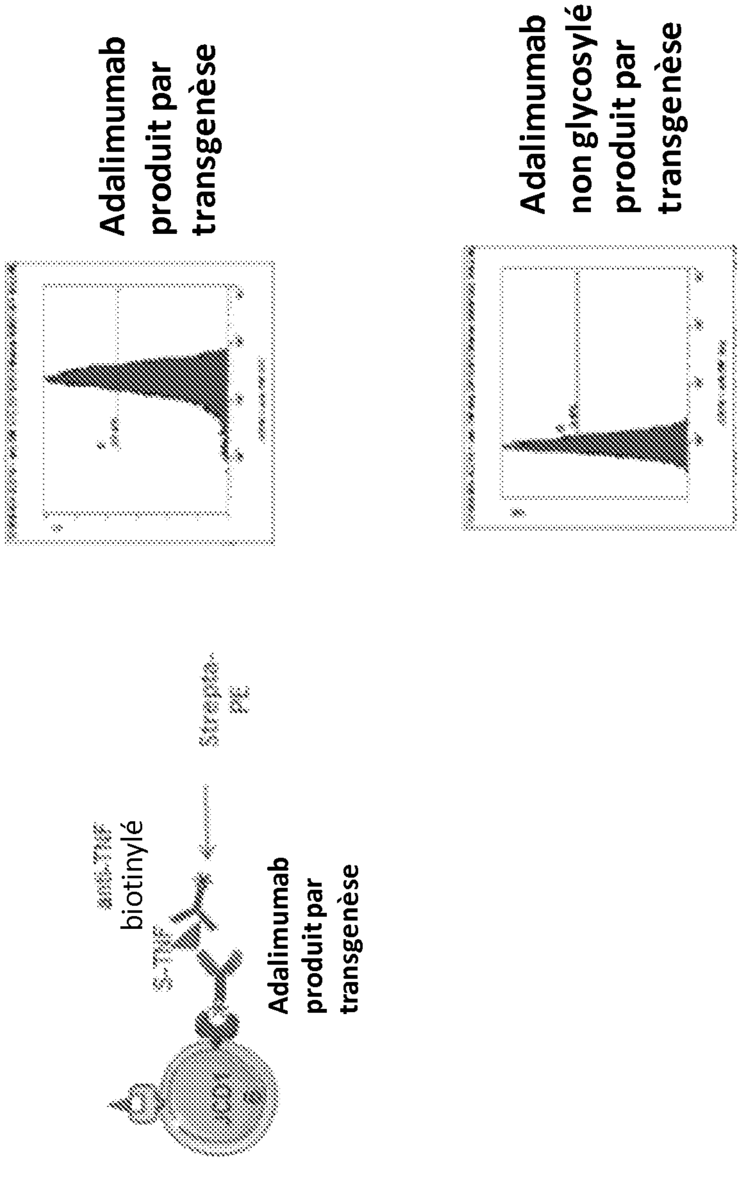
Adalimumab produit par transgenèse
Adalimumab non glycosylé produit par transgenèse
biotinylé
Adalimumab produit par transgenèse

FIGURE 13

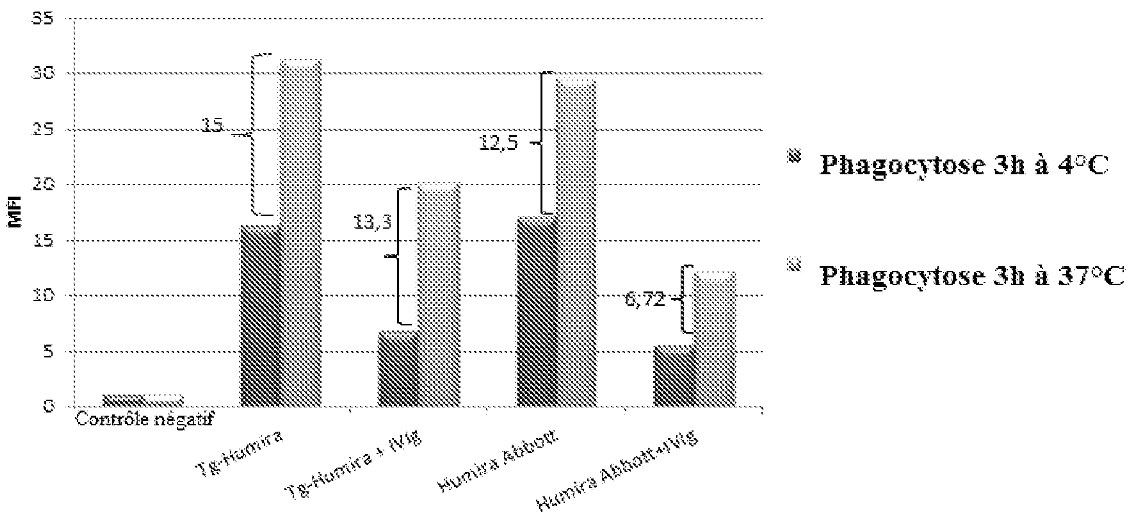
MFI
35
30
25
20
15
10
5
0
15
13,3
12,5
6,72
Contrôle négatif
Tg-Humira
Tg-Humira + IVIg
Humira Abbott
Humira Abbott+IVIg
Phagocytose 3h à 4°C
Phagocytose 3h à 37°C

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 55671
- EP 0562132 A
- US 7700738 B
- US 20100222555 A
- WO 2008145866 A1
- US 8324351 B2
- US 20120088905 A1
- WO 2010088444 A1
- WO 2013011076 A2
- WO 2006040153 A
- WO 2006122786 A
- WO 2003002609 A
- US 5591669 A
- US 5598369 A
- US 5545806 A
- US 5545807 A
- US 6150584 A
- US 4816567 A
- US 6331415 B
- US 6808901 B
- EP 125023 A
- FR 2641468
- US 5204244 A
- US 5202238 A
- US 5225539 A
- US 5585089 A
- EP 0682040 A
- US 5639641 A
- FR 2924431
- WO 2007074496 A
- WO 2006117699 A
- US 7670809 B
- EP 1311670 A
- WO 2009050388 A
- WO 0177181 A
- EP 1071700 A
- US 6602684 B
- EP 1692182 A
- US 2005123546 A
- US 7700321 B
- US 20090317869 A
- US 2010291628 A
- US 20090228994 A
- EP 1500698 A
- EP 1792987 A
- US 7846725 B
- US 7393683 B
- WO 2006133148 A
- WO 200748077 A
- WO 0200879 A
- EP 1176195 A
- US 7214775 B
- US 6994292 B
- US 7425446 B
- US 2010223686 A
- WO 2007099988 A
- EP 1705251 A
- WO 2007048077 A
- US 7928064 B
- US 6090382 A


### Littérature non-brevet citée dans la description


- **STROHL.** *Current Opinion in Biotechnology,* 2009, vol. 20, 685-691
- **SAMBROOK et al.** Molecular Cloning: A Laboratory Manual. Cold Spring Harbor Laboratory Press, 1989
- **LEWIN.** Genes IV. Oxford University Press, 1990
- **ROITT et al.** Immunology. Gower Medical Publishing, 1989
- **WARD et al.** *Nature,* 1989, vol. 341, 544-546
- **BIRD et al.** *Science,* 1988, vol. 242, 423-426
- **HUSTON et al.** *Proc. Natl. Acad. Sci. USA,* 1988, vol. 85, 5879-5883
- **J. GODING.** Monoclonal Antibodies: Principles and Practice. Academic Press, 1983, 98-118
- **HOLLIGER, P. et al.** *Proc. Natl. Acad. Sci. USA,* 1993, vol. 90, 6444-6448
- **POIJAK, R.J. et al.** *Structure,* 1994, vol. 2, 1121-1123
- **WALDMANN.** *Science,* 1991, vol. 252, 1657
- **MALMQUIST M.** *Current Opinion in Immunology,* 1993, vol. 5, 282-286
- **JÖNSSON U et al.** *Biotechniques,* 1991, vol. 11, 620-627
- **WU et al.** *Proc. Natl. Acad. Sci. USA,* 1998, vol. 95, 6037-6042
- **MUTA T et al.** *Nature,* 1994, vol. 368, 70-73
- **RAVETCH ; KINET.** *Annual Review of Immunology,* 1991, vol. 9, 457-492

- **RAGHAVAN et al.** *Annu Rev Cell Dev Biol,* 1996, vol. 12, 181-220
- **RAVETCH et al.** *Annu Rev Immunol,* 2001, vol. 19, 275-290
- **SHAKIB F.** Basic and Clinical Aspects of IgG Subclasses. *Karger,* 1986
- **MUNN DH et al.** *Cancer Research,* 1991, vol. 51, 1117-1123
- **MORRISON et al.** *Proc. Natl. Acad. Sci. U.S.A.,* 1984, vol. 81, 6851-55
- **VERHOEVEN et al.** *BioEssays,* 1988, vol. 8, 74
- **BOULIANNE, G. L. et al.** *Nature,* 1984, vol. 312, 643
- **SUN, L.K. et al.** *Proc. Natl. Acad. Sci. USA,* vol. 84, 214-218
- **BOBRZECKA, K. et al.** *Immunology Letters,* vol. 2, 151-155
- **G.T. STEVENSON et al.** Med. Oncol. & Tumor. *Pharmacother,* 1984, vol. 1 (4), 275-278
- **JONES et al.** *Nature,* 1986, 321-522, 525
- **SINGER et al.** *J. Immun.,* 1993, vol. 150, 2844-2857
- **RIECHMAN et al.** *Nature,* 1988, vol. 323, 326
- **VERHOEYEN et al.** *Science,* 1988, vol. 239, 1534
- **TAMURA et al.** *J Immunol.,* 2000, vol. 164, 1432-41
- **PADLAN et al.** *FASEB J.,* 1995, vol. 9, 133-139
- **DE PASCALIS et al.** *The Journal of Immunology,* 2002, vol. 169, 3076-3084
- **KASHMIRI SYED V.S et al.** *Humanized Antibodies and their Applications,* Mai 2005, vol. 36, 25-34
- **ROGUSKA et al.** *Proc Natl Acad Sci USA,* 1994
- The pharmacology of monoclonal Antibodies. **MARK G. E. et al.** Handbook of Experimental Pharmacology. Springer-Verlag, 1994, vol. 113, 105-134
- **CRAMERI et al.** *Nature medicine,* 1996, vol. 2 (1), 100-2
- **YANG et al.** *J. Mol. Biol.,* 1995, vol. 254, 392-403
- **S.J. CUMBERS et al.** *Nature Biotechnology,* 2002, vol. 20, 1129-1134
- **CHAO, G et al.** *J. Mol. Biol.,* 2004, vol. 342 (2), 539-50